# EUROPEAN PATENT SPECIFICATION

(11) **EP 3 400 443 B1**
(45) Date of publication and mention of the grant of the patent: **16.09.2020**
(21) Application number: 17700023.9
(22) Date of filing: 03.01.2017
(51) Int. Cl.: G01N 33/574

(54) **USE OF PD-1 AND TIM-3 AS A MEASURE FOR CD8+ CELLS IN PREDICTING AND TREATING RENAL CELL CARCINOMA**
VERWENDUNG VON PD-1 UND TIM-3 ZUR MESSUNG VON CD8+ ZELLEN ZUR VORHERSAGE UND BEHANDLUNG VON NIERENZELLKARZINOM
UTILISATION DE PD-1 ET TIM-3 COMME MESURE DE CELLULES CD8+ POUR PRÉDIRE ET TRAITER LE CARCINOME À CELLULES RÉNALES

(30) Priority: 04.01.2016 EP 16305004
(43) Date of publication of application: 14.11.2018
(73) Proprietor: INSERM (Institut National de la Santé et de la Recherche Médicale), 75013 Paris (FR); Université Paris Descartes, 75006 Paris (FR); Assistance Publique-Hôpitaux de Paris (APHP), 75004 Paris (FR)
(72) Inventor: TARTOUR, Eric, 75015 Paris (FR); DARIANE, Charles, 75908 Paris Cedex 15 (FR); GRANIER, Clémence, 75015 Paris (FR); GEY, Alain, 75015 Paris (FR)
(74) Representative: Inserm Transfert
(86) International application number: PCT/EP2017/050088
(87) International publication number: WO 2017/118634

(56) References cited:
- STEFANIE REGINE DANNENMANN ET AL: "Tumor-associated macrophages subvert T-cell function and correlate with reduced survival in clear cell renal cell carcinoma", ONCOIMMUNOLOGY, vol. 2, no. 3, 1 March 2013 (2013-03-01), page e23562, XP055275532, DOI: 10.4161/onci.23562 & Stefanie Regine Dannenmann ET AL: "Supplemental material to: Tumor-associated macrophages subvert T-cell function and correlate with reduced survival in clear cell renal cell carcinoma", OncoImmunology, vol. 2, no. 3, 1 March 2013 (2013-03-01), page e23562, XP055275719, DOI: 10.4161/onci.23562
- S. F. NGIOW ET AL: "A Threshold Level of Intratumor CD8+ T-cell PD1 Expression Dictates Therapeutic Response to Anti-PD1", CANCER RESEARCH, vol. 75, no. 18, 24 July 2015 (2015-07-24) , pages 3800-3811, XP055273363, US ISSN: 0008-5472, DOI: 10.1158/0008-5472.CAN-15-1082
- K. SAKUISHI ET AL: "Targeting Tim-3 and PD-1 pathways to reverse T cell exhaustion and restore anti-tumor immunity", JOURNAL OF EXPERIMENTAL MEDICINE, vol. 207, no. 10, 27 September 2010 (2010-09-27), pages 2187-2194, XP055052551, ISSN: 0022-1007, DOI: 10.1084/jem.20100643
- JING LI ET AL: "Differential expression of PD-1 and Tim-3 marks activation versus exhaustion status of T cells in the tumor microenvironment", JOURNAL FOR IMMUNOTHERAPY OF CANCER, BIOMED CENTRAL LTD, LONDON, UK, vol. 2, no. Suppl 3, 6 November 2014 (2014-11-06), page P220, XP021202488, ISSN: 2051-1426, DOI: 10.1186/2051-1426-2-S3-P220
- Y JIANG ET AL: "T-cell exhaustion in the tumor microenvironment", CELL DEATH AND DISEASE, vol. 6, no. 6, 18 June 2015 (2015-06-18), page e1792, XP055275768, DOI: 10.1038/cddis.2015.162

## Description

### FIELD:

The disclosure is in the field of oncology. In particular, the disclosure relates to methods and pharmaceutical compositions for predicting and treating a subject suffering from renal cell carcinoma.

### BACKGROUND:

Cancer is the result of genetic modifications in normal cells that deregulate cell growth-and-division cycle, cell survival, energy metabolism and cellular migration, causing cells to become autonomous from control signals, reproduce uncontrollably (Hanahan et al., 2011) and establish chronic inflammatory events in the host.

The microenvironment of a developing tumor is a complex tissue composed of proliferating tumor cells, stromal cells, lymphatic endothelial cells, blood vessels, fibroblasts, infiltrating immune cells (myeloid and lymphoid cells) and the extracellular matrix (ECM). It is a unique environment that emerges during tumor and is dominated by the tumor to provide tumor cells with nutrients and oxygen derived from the vascularization networks and growth factors produced by inflammatory and stromal cells for their growth and invasion but also promotes the infiltration of immune cells (Fridman et al., 2014).

The tumoral stroma becomes infiltrated with many other immune cells including neutrophils, eosinophils, basophils, monocytes/macrophages, dendritic cells (DC), natural killer (NK) cells and lymphocytes. Between these, the most predominant cell in the milieu are tumor-associated macrophages (TAMs) and tumor-infiltrate lymphocytes (TILs) populations of CD3+ CD4+ and CD3+ CD8+ T cells presenting mainly a M2 and non-Thl phenotype respectively, when the transition from a precancerous stage to an invasive tumor is set (Whiteside, 2008). Macrophages, granulocytes and myeloid-derived suppressor cells (MDSCs) are found in most cases infiltrating or surrounding tumor beds both in the core and at the invasive front of the tumor (Bingle et al., 2002; Serafini et al., 2006). NK cells can be found in the stroma and invasive margin (Platonova et al., 2011), while immature DC are located in the tumor core or in the stroma (Gabrilovich et al., 1996). Intra-tumoral immunological profiles is correlated with the survival in patients affected by primary ccRCC. More particularly the infiltration of M2 macrophages and Tregs is correlated with a reduced survival (Stefanie Regine Dannememann et al 2013).

TILs distribution is not uniformly between tumors types but mainly all subsets of T cells are found at the invasive margin and at the core of tumor. B cells are mostly found in the tumor invasive margin whereas T cells especially CD8+ T cells are located in the tumor core and the invasive margin where they have a better interaction with tumor cells.

CD8+ T cells responses are necessary for the control of tumors. Upon encounter with antigen through their TCRs, naive CD8+ T cells initiate multiple intracellular signals that lead to a cellular response. This includes changes in cell surface phenotype, high proliferation, acquisition of effector functions (such as cytokine secretion and cytotoxicity) and altered survival requirements to constitute the memory pool.

Once CD8+ T cells acquire their effector functions, a range of effector molecules produce and mediate the defense against cancer cells through the direct cytolysis of target cells (mediated by perforin and granzyme molecules), Fas signaling, secretion of cytokines (TNF-α, IFN-γ) and chemokines that attract inflammatory cells (Harty et al., 2000; Wong and Pamer, 2003).

In tumors such as colorectal cancer, lung cancer and ovarian carcinoma, high densities of intratumoral memory (CD45RO+) cells and CD8+ T-cells are correlated with a favorable prognosis.

Kidney cancer also called "renal cancer," or "renal cell carcinoma" refers to cancer that has arisen from the kidney. Renal cell carcinoma (RCC), also known as renal cell cancer or renal cell adenocarcinoma, is by far the most common type of kidney cancer and originates in the lining of the proximal convoluted tubule. About 9 out of 10 kidney cancers are renal cell carcinomas. More specifically, RCC encompasses several relatively common histologic subtypes: clear cell renal cell carcinoma, papillary (chromophil), chromophobe, collecting duct carcinoma, and medullary carcinoma. Clear cell renal cell carcinoma (ccRCC) is the most common subtype of RCC. Incidence of ccRCC is increasing, comprising 80% of localized disease and more than 90% of metastatic disease.

Immunotherapy is a new class of cancer treatment that works to harness the innate powers of the immune system to fight cancer. Because of the immune system's unique properties, these therapies may hold greater potential than current treatment approaches to fight cancer more powerfully, to offer longer-term protection against the disease, to come with fewer side effects, and to benefit more patients with more cancer types. Different immunotherapy strategies exist to treat kidney cancer: 1) cytokines which are used most often to treat kidney cancer, are interleukin-2 (IL-2) and interferon-alpha. Both cytokines can cause kidney cancers to shrink in a small percentage of patients. Giving high doses of IL-2 seems to offer the best chance of shrinking the cancer, but this can cause serious side effects, so it is not used in people who are in poor overall health to begin with. Special care is needed to recognize and treat these side effects; 2) Treatment with Interferon has less serious side effects than IL-2, but it does not seem to be as effective when used by itself. It is more often used in combination with the targeted drug bevacizumab (Avastin); 3) an important part of the immune system is its ability to keep itself from attacking normal cells in the body. To do this, it uses "checkpoints," which are molecules on immune cells that need to be turned on (or off) to start an immune response. Cancer cells sometimes use these checkpoints to avoid being attacked by the immune system. But newer drugs that target these checkpoints hold a lot of promise as cancer treatments. Drugs that target PD-1, a protein on immune system cells called programmed cell death 1, T cells that normally helps keep these cells from attacking other cells in the body. By blocking PD-1, the drug boosts the immune response against cancer cells. However, in RCC cancer, the treatment based on the inhibition of PD-1 leads only about 30% clinical responses in cancer patients. S.F. Ngiow et al 2015 have shown that intratumor Tregs are partly responsible for the development of anti-PD1-resistant tumors and PDlhi CD8⁺ T cells. Co-expression of distinct inhibitory receptors has been associated with greater T-cell exhaustion (Sakuishi et al 2010, Jing et al 2014 and Jiang et al 2015).A recent clinical trial on phase III has shown that patients with advanced renal-cell carcinoma who had received previous antiangiogenic treatment had longer survival with nivolumab treatment than with everolimus treatment (Motzer et al 2015). In this trial, the expression of PD-L1 was not correlated with treatment response, thus there is a need to identify and validate others biomarkers of treatment response.

Thus, there is a need to find a treatment strategy which allows to have few side effects and avoid relapses.

### SUMMARY:

The disclosure relates to:
A method for predicting the survival time of a subject suffering from renal cell carcinoma comprising the steps of: i) quantifying the percent of CD8+ T cells co-expressing PD-1 and Tim-3 in a tumor tissue sample obtained from the subject, ii) comparing the percent quantified at step i) with its corresponding predetermined reference value and iii) concluding that the subject will have a short survival time when the percent of CD8+ T cells co-expressing PD-1 and Tim-3 is higher than its corresponding predetermined reference value or concluding that the subject will have a long survival time when the percent of CD8+ T cells co-expressing PD-1 and Tim-3 is lower than its corresponding predetermined reference value.

The method of the disclosure wherein, the quantification of percent of CD8+ T cells co-expressing PD-1 and Tim-3 is determined by Immunohistochemistry (IHC).

The method of the disclosure wherein, the quantification of percent of CD8+ T cells co-expressing PD-1 and Tim-3 is determined by an automatized microscope.

A method for determining whether a subject suffering from a renal cell carcinoma will achieve a response with an immune-checkpoint inhibitor comprising the steps of i) quantifying the percent of CD8+ T cells co-expressing PD-1 and Tim-3 in a tumor tissue sample obtained from the subject treated with an immune-checkpoint inhibitor, ii) comparing the percent CD8+ T cells co-expressing PD-1 and Tim-3 quantified at step i) with its corresponding predetermined reference values and iii) concluding that the subject will not respond to the treatment when the percent of CD8+ T cells co-expressing PD-1 and Tim-3 is higher than its corresponding predetermined reference value or concluding that the subject will respond to the treatment when the percent of CD8+ T cells co-expressing PD-1 and Tim-3 is lower than its corresponding predetermined reference value.

The method of the disclosure, wherein the immune checkpoint inhibitor is an antibody.

The method of the disclosure, wherein the immune checkpoint inhibitor is a monoclonal antibody.

The method of the disclosure, wherein the immune checkpoint inhibitor is an anti-PD-1 antibody.

The method of the disclosure, wherein the immune checkpoint inhibitor is an anti-PD-L1 antibody.

The method of the disclosure, wherein the immune checkpoint inhibitor is an anti-PD-L2 antibody.

The method of the disclosure, wherein the immune checkpoint inhibitor is an anti-Tim-3 antibody.

The method of the disclosure, wherein the immune checkpoint inhibitor is a small organic molecule.

The method of the disclosure, wherein the immune checkpoint inhibitor is an aptamer.

### DETAILED DESCRIPTION:

Using a novel spectral multi-immunofluorescence in situ imaging technology, the inventors of the present disclosure showed that the clinical significance of PD-1 on CD8+T cells differs whether it was co-expressed or not with Tim-3. Indeed, they showed that renal cell carcinoma patients which co-expressed PD-1 and Tim-3 had a more aggressive phenotype defined by high Fuhrman grade and tumor of larger size and advanced TNM and UISS score.

Accordingly, the present disclosure relates to a method for predicting the survival time of a subject suffering from renal cell carcinoma comprising the steps of: i) quantifying the percent of CD8+ T cells co-expressing PD-1 and Tim-3 in a tumor tissue sample obtained from the subject, ii) comparing the percent quantified at step i) with its corresponding predetermined reference values and iii) concluding that the subject will have a short survival time when the percent of CD8+ T cells co-expressing PD-1 and Tim-3 is higher than its corresponding predetermined reference value or concluding that the subject will have a long survival time when the percent of CD8+ T cells co-expressing PD-1 and Tim-3 is lower than its corresponding predetermined reference value.

As used herein, the terms "kidney cancer," "renal cancer," or "renal cell carcinoma" refer to cancer that has arisen from the kidney. The terms "renal cell cancer" or "renal cell carcinoma" (RCC), as used herein, refer to cancer which originates in the lining of the proximal convoluted tubule. More specifically, RCC encompasses several relatively common histologic subtypes: clear cell renal cell carcinoma, papillary (chromophil), chromophobe, collecting duct carcinoma, and medullary carcinoma. Clear cell renal cell carcinoma (ccRCC) is the most common subtype of RCC.

The method is particularly suitable for predicting the duration of the overall survival (OS), progression-free survival (PFS) and/or the disease-free survival (DFS) of the cancer subject. Those of skill in the art will recognize that OS survival time is generally based on and expressed as the percentage of people who survive a certain type of cancer for a specific amount of time. Cancer statistics often use an overall five-year survival rate. In general, OS rates do not specify whether cancer survivors are still undergoing treatment at five years or if they have become cancer-free (achieved remission). DSF gives more specific information and is the number of people with a particular cancer who achieve remission. Also, progression-free survival (PFS) rates (the number of people who still have cancer, but their disease does not progress) include people who may have had some success with treatment, but the cancer has not disappeared completely. As used herein, the expression "short survival time" indicates that the subject will have a survival time that will be lower than the median (or mean) observed in the general population of subjects suffering from said cancer. When the subject will have a short survival time, it is meant that the subject will have a "poor prognosis". Inversely, the expression "long survival time" indicates that the subject will have a survival time that will be higher than the median (or mean) observed in the general population of subjects suffering from said cancer. When the subject will have a long survival time, it is meant that the subject will have a "good prognosis".

As used herein, the term "tumor tissue sample" has its general meaning in the art and encompasses pieces or slices of tissue that have been removed including following a surgical tumor resection. The tumor tissue sample can be subjected to a variety of well-known post-collection preparative and storage techniques (e.g., fixation, storage, freezing, etc.) prior to determining the cell densities. Typically the tumor tissue sample is fixed in formalin and embedded in a rigid fixative, such as paraffin (wax) or epoxy, which is placed in a mould and later hardened to produce a block which is readily cut. Thin slices of material can be then prepared using a microtome, placed on a glass slide and submitted e.g. to immunohistochemistry (IHC) (using an IHC automate such as BenchMark® XT or Autostainer Dako, for obtaining stained slides). The tumour tissue sample can be used in microarrays, called as tissue microarrays (TMAs). TMA consists of paraffin blocks in which up to 1000 separate tissue cores are assembled in array fashion to allow multiplex histological analysis. This technology allows rapid visualization of molecular targets in tissue specimens at a time, either at the DNA, RNA or protein level. TMA technology is described in WO2004000992, US8068988, Olli et al 2001 Human Molecular Genetics, Tzankov et al 2005, Elsevier; Kononen et al 1198; Nature Medicine.

As used herein "CD8 + T cells" has its general meaning in the art and refers to a subset of T cells which express CD8 on their surface. They are MHC class I-restricted, and function as cytotoxic T cells. "CD8 T+ cells" are also called CD8 T cells are called cytotoxic T lymphocytes (CTL), T-killer cell, cytolytic T cells, or killer T cells. CD8 antigens are members of the immunoglobulin supergene family and are associative recognition elements in major histocompatibility complex class I-restricted interactions. In the context of the disclosure, CD8+T cells are considered as TILS, for tumor-infiltrating lymphocytes. This kind of lymphocytes are present in tumors. They are implicated in killing tumor cells, and in the art, their presence in tumors is often associated with a better clinical outcomes.

As used herein, the term "CD8+T cells co-expressing PD-1 and Tim-3" refers to CD 8+ T cells which express at their surface both PD-1 and Tim-3.

PD-1 refers to programmed cell death protein 1 and is expressed on T cells, B cells, and macrophages. The ligands for PD-1 are the B7 family members PD-L1 (B7-H1) and PD-L2 (B7-DC). PD-L1 refers to programmed death-ligand 1 which is present on tumor cells, in particular on kidney cancer cells.

Tim-3 is a member of the T cell immunoglobulin and mucin domain (Tim) family, which encompasses a group of type I transmembrane proteins expressed by both innate and adaptive cell types within the immune system. TIM gene family consists of eight members (TIM-1-8) located on chromosome 11B1.1 in mouse, and three members (TIM-1, TIM-3, and TIM-4) located on chromosome 5q33.2 in human. Binding of TIM-3 to a protein ligand (e.g., galectin-9) can inhibit the Th1 response via mechanism of apoptosis induction, and therefore lead to such as induction of peripheral tolerance.

In particular the quantification of percent of CD8+ T cells co-expressing PD-1 and Tim-3 is determined by Immunohistochemistry (IHC).

For example, the quantification of percent of CD8+ T cells co-expressing PD-1 and Tim-3 is performed by contacting the tumor tissue sample with binding partners (e.g. antibodies) specific for the cell surface markers of said cells. Typically, the quantification of density of CD8+T cells co-expressing PD-1 and Tim-3 is performed by contacting the tumor tissue sample with a binding partner (e.g. antibodies) specific for CD8, PD-1 and Tim-3.

Typically, the percent of CD8+T cells co-expressing PD-1 and Tim-3 consist of the percentage of the specific cells per total of CD8+ T cells (set at 100%). In particular, the number of these cells that are counted per one unit of surface area of tissue sample, e.g. as the number of cells that are counted per mm² of surface area of tumor tissue sample.

Immunohistochemistry typically includes the following steps i) fixing the tumor tissue sample with formalin, ii) embedding said tumor tissue sample in paraffin, iii) cutting said tumor tissue sample into sections for staining, iv) incubating said sections with the binding partner specific for the marker, v) rinsing said sections, vi) incubating said section with a secondary antibody typically biotinylated and vii) revealing the antigen-antibody complex typically with avidin-biotin-peroxidase complex. Accordingly, the tumor tissue sample is firstly incubated with the binding partners, such as anti-CD8 antibody, anti-PD-1 antibody and anti-Tim-3 antibody. After washing, the labeled antibodies that are bound to marker of interest are revealed by the appropriate technique, depending of the kind of label is borne by the labeled antibody, e.g. radioactive, fluorescent or enzyme label. Multiple labelling can be performed simultaneously. Alternatively, the method of the present disclosure may use a secondary antibody coupled to an amplification system (to intensify staining signal) and enzymatic molecules. Such coupled secondary antibodies are commercially available, e.g. from Dako, EnVision system. Counterstaining may be used, e.g. Hematoxylin & Eosin, DAPI, Hoechst. Other staining methods may be accomplished using any suitable method or system as would be apparent to one of skill in the art, including automated, semi-automated or manual systems. For example, one or more labels can be attached to the antibody, thereby permitting detection of the target protein (i.e the marker). Exemplary labels include radioactive isotopes, fluorophores, ligands, chemiluminescent agents, enzymes, and combinations thereof. In particular In particularThe disclosure discloses that the label is a quantum dot. Non-limiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke J R (1972) Annu. Rev. Biochem. 41:843-868. Affinity ligands can also be labeled with enzymes (e.g. horseradish peroxidase, alkaline phosphatase, beta-lactamase), radioisotopes (e.g. ³H, ¹⁴C, ³²P, ³⁵S or ¹²⁵I) and particles (e.g. gold). The different types of labels can be conjugated to an affinity ligand using various chemistries, e.g. the amine reaction or the thiol reaction. However, other reactive groups than amines and thiols can be used, e.g. aldehydes, carboxylic acids and glutamine. Various enzymatic staining methods are known in the art for detecting a protein of interest. For example, enzymatic interactions can be visualized using different enzymes such as peroxidase, alkaline phosphatase, or different chromogens such as DAB, AEC or Fast Red. In other examples, the antibody can be conjugated to peptides or proteins that can be detected via a labeled binding partner or antibody. In an indirect IHC assay, a secondary antibody or second binding partner is necessary to detect the binding of the first binding partner, as it is not labeled. The resulting stained specimens are each imaged using a system for viewing the detectable signal and acquiring an image, such as a digital image of the staining. Methods for image acquisition are well known to one of skill in the art. For example, once the sample has been stained, any optical or non-optical imaging device can be used to detect the stain or biomarker label, such as, for example, upright or inverted optical microscopes, scanning confocal microscopes, cameras, scanning or tunneling electron microscopes, canning probe microscopes and imaging infrared detectors. In some examples, the image can be captured digitally. The obtained images can then be used for quantitatively or semi-quantitatively determining the amount of the marker in the sample, or the absolute number of cells positive for the maker of interest, or the surface of cells positive for the maker of interest. Various automated sample processing, scanning and analysis systems suitable for use with IHC are available in the art. Such systems can include automated staining and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, e.g., the CAS-200 system (Becton, Dickinson & Co.). In particular, detection can be made manually or by image processing techniques involving computer processors and software. Using such software, for example, the images can be configured, calibrated, standardized and/or validated based on factors including, for example, stain quality or stain intensity, using procedures known to one of skill in the art (see e.g., published U.S. Patent Publication No. US20100136549). The image can be quantitatively or semi-quantitatively analyzed and scored based on staining intensity of the sample. Quantitative or semi-quantitative histochemistry refers to method of scanning and scoring samples that have undergone histochemistry, to identify and quantitate the presence of the specified biomarker (i.e. the marker). Quantitative or semi-quantitative methods can employ imaging software to detect staining densities or amount of staining or methods of detecting staining by the human eye, where a trained operator ranks results numerically. For example, images can be quantitatively analyzed using a pixel count algorithms and tissue recognition pattern (e.g. Aperio Spectrum Software, Automated QUantitatative Analysis platform (AQUA® platform), or Tribvn with Ilastic and Calopix software), and other standard methods that measure or quantitate or semi-quantitate the degree of staining; see e.g., U.S. Pat. No. 8,023,714; U.S. Pat. No. 7,257,268; U.S. Pat. No. 7,219,016; U.S. Pat. No. 7,646,905; published U.S. Patent Publication No. US20100136549 and 20110111435; Camp et al. (2002) Nature Medicine, 8:1323-1327; Bacus et al. (1997) Analyt Quant Cytol Histol, 19:316-328). A ratio of strong positive stain (such as brown stain) to the sum of total stained area can be calculated and scored. The amount of the detected biomarker (i.e. the marker) is quantified and given as a percentage of positive pixels and/or a score. For example, the amount can be quantified as a percentage of positive pixels. In some examples, the amount is quantified as the percentage of area stained, e.g., the percentage of positive pixels. For example, a sample can have at least or about at least or about 0, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 40%, 45%, 50%, 55%, 60%, 65%, 70%, 75%, 80%, 85%, 90%, 95% or more positive pixels as compared to the total staining area. For example, the amount can be quantified as an absolute number of cells positive for the maker of interest. In particular a score is given to the sample that is a numerical representation of the intensity or amount of the histochemical staining of the sample, and represents the amount of target biomarker (e.g., the marker) present in the sample. Optical density or percentage area values can be given a scaled score, for example on an integer scale. Thus, in particular, the method of the present disclosure comprises the steps consisting in i) providing one or more immunostained slices of tissue section obtained by an automated slide-staining system by using a binding partner capable of selectively interacting with the marker (e.g. an antibody as above described), ii) proceeding to digitalisation of the slides of step i).by high resolution scan capture, iii) detecting the slice of tissue section on the digital picture iv) providing a size reference grid with uniformly distributed units having a same surface, said grid being adapted to the size of the tissue section to be analyzed, and v) detecting, quantifying and measuring intensity or the absolute number of stained cells in each unit whereby the number or the density of cells stained of each unit is assessed.

In particular, the quantification of percent of CD8+ T cells co-expressing PD-1 and Tim-3 is determined by an automatized microscope which allows measurement of morphometric and fluorescence characteristics in the different cell compartments (membrane/ cytoplasm/ nuclei) and quantifying preciously the percent of interest cells. Briefly the quantification of percent of CD8+ T cells co-expressing PD-1 and Tim-3 is performed by following steps: i) providing tissue microarray (TMA) containing RCC samples, ii) TMA samples are stained with anti-CD3, anti-CD8, anti-PD-1 and Tim-3 antibodies, iii) the samples are further stained with an epithelial cell marker to assist in automated segmentation of tumour and stroma, iv) TMA slides are then scanned using a multispectral imaging system, v) the scanned images are processed using an automated image analysis software (e.g.Perkin Elmer Technology) which allows the detection and segmentation of specific tissues through powerful pattern recognition algorithms, a machine-learning algorithm is trained to segment tumor from stroma and identify cells labelled; vi) the percent of CD8+ T cells co-expressing PD-1 and Tim-3 within the tumour areas is calculated; vii) a pathologist rates lymphocytes percentage; and vii) manual and automated scoring are compared with survival time of the subj ect.

As used herein, the term "the predetermined reference value" refers to a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. For example, retrospective measurement of cell densities in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after quantifying the cell density in a group of reference, one can use algorithmic analysis for the statistic treatment of the measured densities in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER. SAS, CREATE-ROC.SAS, GB STAT VI0.0 (Dynamic Microsystems, Inc. Silver Spring, Md., USA), etc.

In particular, the predetermined reference value is determined by carrying out a method comprising the steps of
a) providing a collection of tumor tissue samples from subject suffering from a RCC;
b) providing, for each tumor tissue sample provided at step a), information relating to the actual clinical outcome for the corresponding subject (i.e. the duration of the disease-free survival (DFS) and/or the overall survival (OS));
c) providing a serial of arbitrary quantification values;
d) quantifying the percentage of CD8+ T cells for each tumor tissue sample contained in the collection provided at step a);
e) classifying said tumor tissue samples in two groups for one specific arbitrary quantification value provided at step c), respectively: (i) a first group comprising tumor tissue samples that exhibit a quantification value for level that is lower than the said arbitrary quantification value contained in the said serial of quantification values; (ii) a second group comprising tumor tissue samples that exhibit a quantification value for said level that is higher than the said arbitrary quantification value contained in the said serial of quantification values; whereby two groups of tumor tissue samples are obtained for the said specific quantification value, wherein the tumor tissue samples of each group are separately enumerated;
f) calculating the statistical significance between (i) the quantification value obtained at step e) and (ii) the actual clinical outcome of the subjects from which tumor tissue samples contained in the first and second groups defined at step f) derive;
g) reiterating steps f) and g) until every arbitrary quantification value provided at step d) is tested;
h) setting the said predetermined reference value as consisting of the arbitrary quantification value for which the highest statistical significance (most significant P-value obtained with a log-rank test, significance when P<0.05) has been calculated at step g).

For example the cell density has been assessed for 100 tumor tissue samples of 100 subjects. The 100 samples are ranked according to the cell density. Sample 1 has the highest density and sample 100 has the lowest density. A first grouping provides two subsets: on one side sample Nr 1 and on the other side the 99 other samples. The next grouping provides on one side samples 1 and 2 and on the other side the 98 remaining samples etc., until the last grouping: on one side samples 1 to 99 and on the other side sample Nr 100. According to the information relating to the actual clinical outcome for the corresponding cancer subject, Kaplan-Meier curves are prepared for each of the 99 groups of two subsets. Also for each of the 99 groups, the p value between both subsets was calculated (log-rank test). The predetermined reference value is then selected such as the discrimination based on the criterion of the minimum P-value is the strongest. In other terms, the cell density corresponding to the boundary between both subsets for which the P-value is minimum is considered as the predetermined reference value. It should be noted that the predetermined reference value is not necessarily the median value of cell densities. Thus in particular, the predetermined reference value thus allows discrimination between a poor and a good prognosis with respect to DFS and OS for a subject. Practically, high statistical significance values (e.g. low P values) are generally obtained for a range of successive arbitrary quantification values, and not only for a single arbitrary quantification value. Thus, in one alternative embodiment of the disclosure, instead of using a definite predetermined reference value, a range of values is provided. Therefore, a minimal statistical significance value (minimal threshold of significance, e.g. maximal threshold P value) is arbitrarily set and a range of a plurality of arbitrary quantification values for which the statistical significance value calculated at step g) is higher (more significant, e.g. lower P-value) are retained, so that a range of quantification values is provided. This range of quantification values includes a "cut-off" value as described above. For example, according to this specific embodiment of a "cut-off" value, the outcome can be determined by comparing the cell density with the range of values which are identified. In particular, a cut-off value thus consists of a range of quantification values, e.g. centered on the quantification value for which the highest statistical significance value is found (e.g. generally the minimum P-value which is found).

In a further embodiment, the present disclosure relates to a method for determining whether a subject suffering from renal cell carcinoma will achieve a response with an immune-checkpoint inhibitor. In particular, the present disclosure relates to a method for determining whether a subject suffering from a renal cell carcinoma will achieve a response with an immune-checkpoint inhibitor comprising the steps of i) quantifying the percent of CD8+ T cells co-expressing PD-1 and Tim-3 in a tumor tissue sample obtained from the subject treated with an immune-checkpoint inhibitor, ii) comparing the percent CD8+ T cells co-expressing PD-1 and Tim-3 quantified at step i) with its corresponding predetermined reference values and iii) concluding that the subject will not respond to the treatment when the percent of CD8+ T cells co-expressing PD-1 and Tim-3 is higher than its corresponding predetermined reference value or concluding that the subject will respond to the treatment when the percent of CD8+ T cells co-expressing PD-1 and Tim-3 is lower than its corresponding predetermined reference value.

As used herein, the term "respond" refers when the survival time of the subject is increased with a treatment. In particular, in the context of the disclosure, the term "respond" refers to the ability of the immune system to decrease tumour masse, thus, the subject presents a clinical improvement compared to the subject who does not receive the treatment. The said subject is considered as a "responder" to the treatment. The term "not respond" refers to a subject who does not present any clinical improvement to the treatment with an immune checkpoint inhibitor treatment. This subject is considered as a "non-responder" to the treatment.

As used herein, the term "immune checkpoint inhibitor" refers to molecules that totally or partially reduce, inhibit, interfere with or modulate one or more immune checkpoint proteins.

As used herein, the term "immune checkpoint protein" has its general meaning in the art and refers to a molecule that is expressed by T cells in that either turn up a signal (stimulatory checkpoint molecules) or turn down a signal (inhibitory checkpoint molecules). Immune checkpoint molecules are recognized in the art to constitute immune checkpoint pathways similar to the CTLA-4 and PD-1 dependent pathways (see e.g. Pardoll, 2012. Nature Rev Cancer 12:252-264; Mellman et al. , 2011. Nature 480:480- 489). Examples of stimulatory checkpoint include CD27 CD28 CD40, CD122, CD137, OX40, GITR, and ICOS. Examples of inhibitory checkpoint molecules include A2AR, B7-H3, B7-H4, BTLA, CTLA-4, CD277, IDO, KIR, PD-1, LAG-3, TIM-3 and VISTA. The Adenosine A2A receptor (A2AR) is regarded as an important checkpoint in cancer therapy because adenosine in the immune microenvironment, leading to the activation of the A2a receptor, is negative immune feedback loop and the tumor microenvironment has relatively high concentrations of adenosine. B7-H3, also called CD276, was originally understood to be a co-stimulatory molecule but is now regarded as co-inhibitory. B7-H4, also called VTCN1, is expressed by tumor cells and tumor-associated macrophages and plays a role in tumour escape. B and T Lymphocyte Attenuator (BTLA) and also called CD272, has HVEM (Herpesvirus Entry Mediator) as its ligand. Surface expression of BTLA is gradually downregulated during differentiation of human CD8+ T cells from the naive to effector cell phenotype, however tumor-specific human CD8+ T cells express high levels of BTLA. CTLA-4, Cytotoxic T-Lymphocyte-Associated protein 4 and also called CD152. Expression of CTLA-4 on Treg cells serves to control T cell proliferation. IDO, Indoleamine 2,3-dioxygenase, is a tryptophan catabolic enzyme. A related immune-inhibitory enzymes. Another important molecule is TDO, tryptophan 2,3-dioxygenase. IDO is known to suppress T and NK cells, generate and activate Tregs and myeloid-derived suppressor cells, and promote tumour angiogenesis. KIR, Killer-cell Immunoglobulin-like Receptor, is a receptor for MHC Class I molecules on Natural Killer cells. LAG3, Lymphocyte Activation Gene-3, works to suppress an immune response by action to Tregs as well as direct effects on CD8+ T cells. PD-1, Programmed Death 1 (PD-1) receptor, has two ligands, PD-L1 and PD-L2. This checkpoint is the target of Merck & Co.'s melanoma drug Keytruda, which gained FDA approval in September 2014. An advantage of targeting PD-1 is that it can restore immune function in the tumor microenvironment. TIM-3, short for T-cell Immunoglobulin domain and Mucin domain 3, expresses on activated human CD4+ T cells and regulates Th1 and Th17 cytokines. TIM-3 acts as a negative regulator of Th1/Tc1 function by triggering cell death upon interaction with its ligand, galectin-9. VISTA, Short for V-domain Ig suppressor of T cell activation, VISTA is primarily expressed on hematopoietic cells so that consistent expression of VISTA on leukocytes within tumors may allow VISTA blockade to be effective across a broad range of solid tumors.

These proteins are responsible for co-stimulatory or inhibitory interactions of T-cell responses. Thus, immune checkpoint proteins regulate and maintain self-tolerance and the duration and amplitude of physiological immune responses.

Tumor cells often take advantage of these checkpoints to escape detection by the immune system. Thus, inhibiting a checkpoint protein on the immune system may enhance the anti-tumor T-cell response.

In particular, an immune checkpoint inhibitor refers to any compound inhibiting the function of an immune checkpoint protein. Inhibition includes reduction of function and full blockade.

In particular, the immune checkpoint inhibitor could be an antibody, synthetic or native sequence peptides, small molecules or aptamers which bind to the immune checkpoint proteins and their ligands.

In particular the immune checkpoint inhibitor is an antibody.

As used herein, the term "antibody" is used in the broadest sense and specifically covers monoclonal antibodies, polyclonal antibodies, multispecific antibodies (e.g. bispecific antibodies) formed from at least two intact antibodies, and antibody fragments so long as they exhibit the desired biological activity. In natural antibodies, two heavy chains are linked to each other by disulfide bonds and each heavy chain is linked to a light chain by a disulfide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The term includes antibody fragments that comprise an antigen binding domain such as Fab', Fab, F(ab')2, single domain antibodies (DABs), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); BiTE (Bispecific T-cell Engager, scFv-scFv tandems to attract T cells); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affinity ReTargeting"); small antibody mimetics comprising one or more CDRs and the like. The techniques for preparing and using various antibody-based constructs and fragments are well known in the art (see Kabat et al., 1991, specifically incorporated herein by reference). Diabodies, in particular, are further described in EP 404, 097 and WO 93/1 1 161; whereas linear antibodies are further described in Zapata et al. (1995). Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001 ; Reiter et al., 1996; and Young et al., 1995 further describe and enable the production of effective antibody fragments. In particular, the antibody is a "chimeric" antibody as described in U.S. Pat. No. 4,816,567. In particular, the antibody is a humanized antibody, such as described U.S. Pat. Nos. 6,982,321 and 7,087,409. In particular, the antibody is a human antibody. A "human antibody" such as described in US 6,075,181 and 6,150,584. In particular, the antibody is a single domain antibody such as described in EP 0 368 684, WO 06/030220 and WO 06/003388.

In particular, the immune checkpoint inhibitor is a monoclonal antibody.

Monoclonal antibodies can be prepared and isolated using any technique that provides for the production of antibody molecules by continuous cell lines in culture. Techniques for production and isolation include but are not limited to the hybridoma technique, the human B-cell hybridoma technique and the EBV-hybridoma technique. Typically, antibodies are directed against A2AR, B7-H3, B7-H4, BTLA, CTLA-4, CD277, IDO, KIR, PD-1, LAG-3, TIM-3 or VISTA. In particular, the immune checkpoint inhibitor is an anti-PD-1 antibody such as described in WO2011082400, WO2006121168, WO2015035606, WO2004056875, WO2010036959, WO2009114335, WO2010089411, WO2008156712, WO2011110621, WO2014055648 and WO2014194302. Examples of anti-PD-1 antibodies which are commercialized: Nivolumab (Opdivo®, BMS), Pembrolizumab (also called Lambrolizumab, KEYTRUDA® or MK-3475, MERCK). In particular, the immune checkpoint inhibitor is an anti-PD-L1 antibody such as described in WO2013079174, WO2010077634, WO2004004771, WO2014195852, WO2010036959, WO2011066389, WO2007005874, WO2015048520, US8617546 and WO2014055897. Examples of anti-PD-L1 antibodies which are on clinical trial: Atezolizumab (MPDL3280A, Genentech/Roche), Durvalumab (AZD9291, AstraZeneca), Avelumab (also known as MSB0010718C, Merck) and BMS-936559 (BMS). In particular, the immune checkpoint inhibitor is an anti-PD-L2 antibody such as described in US7709214, US7432059 and US8552154. In the context of the disclosure, the immune checkpoint inhibitor inhibits Tim-3 or its ligand.

In particular, the immune checkpoint inhibitor is an anti-Tim-3 antibody such as described in WO03063792, WO2011155607, WO2015117002, WO2010117057 and WO2013006490.

In particular, the immune checkpoint inhibitor is a small organic molecule.

The term "small organic molecule" as used herein, refers to a molecule of a size comparable to those organic molecules generally used in pharmaceuticals. The term excludes biological macro molecules (e. g. proteins, nucleic acids, etc.). Typically, small organic molecules range in size up to about 5000 Da, more preferably up to 2000 Da, and most preferably up to about 1000 Da.

Typically, the small organic molecules interfere with transduction pathway of A2AR, B7-H3, B7-H4, BTLA, CTLA-4, CD277, IDO, KIR, PD-1, LAG-3, TIM-3 or VISTA.

In particular, small organic molecules interfere with transduction pathway of PD-1 and Tim-3. For example, they can interfere with molecules, receptors or enzymes involved in PD-1 and Tim-3 pathway.

In particular, the small organic molecules interfere with Indoleamine-pyrrole 2,3-dioxygenase (IDO) inhibitor. IDO is involved in the tryptophan catabolism (Liu et al 2010, Vacchelli et al 2014, Zhai et al 2015). Examples of IDO inhibitors are described in WO 2014150677. Examples of IDO inhibitors include without limitation 1-methyl-tryptophan (IMT), β-(3-benzofuranyl)-alanine, β-(3-benzo(b)thienyl)-alanine), 6-nitro-tryptophan, 6-fluoro-tryptophan, 4-methyl-tryptophan, 5 -methyl tryptophan, 6-methyl-tryptophan, 5-methoxy-tryptophan, 5 -hydroxy-tryptophan, indole 3-carbinol, 3,3'- diindolylmethane, epigallocatechin gallate, 5-Br-4-Cl-indoxyl 1,3-diacetate, 9- vinylcarbazole, acemetacin, 5-bromo-tryptophan, 5-bromoindoxyl diacetate, 3- Amino-naphtoic acid, pyrrolidine dithiocarbamate, 4-phenylimidazole a brassinin derivative, a thiohydantoin derivative, a β-carboline derivative or a brassilexin derivative. In particular the IDO inhibitor is selected from 1-methyl-tryptophan, β-(3-benzofuranyl)-alanine, 6-nitro-L-tryptophan, 3-Amino-naphtoic acid and β-[3-benzo(b)thienyl] -alanine or a derivative or prodrug thereof.

In particular, the inhibitor of IDO is Epacadostat, (INCB24360, INCB024360) has the following chemical formula in the art and refers to -N-(3-bromo-4-fluorophenyl)-N'-hydroxy-4-{[2-(sulfamoylamino)-ethyl]amino}-1,2,5-oxadiazole-3 carboximidamide :

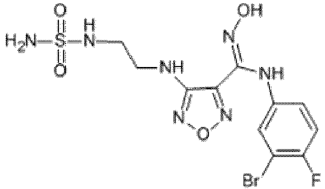

In particular, the inhibitor is BGB324, also called R428, such as described in WO2009054864, refers to 1H-1,2,4-Triazole-3,5-diamine, 1-(6,7-dihydro-5H-benzo[6,7]cyclohepta[1,2-c]pyridazin-3-yl)-N3-[(7S)-6,7,8,9-tetrahydro-7-(1-pyrrolidinyl)-5H-benzocyclohepten-2-yl]- and has the following formula in the art:

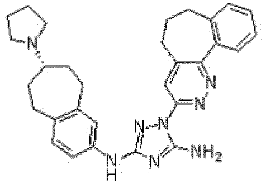

In particular, the inhibitor is CA-170 (or AUPM-170): an oral, small molecule immune checkpoint antagonist targeting programmed death ligand-1 (PD-L1) and V-domain Ig suppressor of T cell activation (VISTA) (Liu et al 2015). Preclinical data of CA-170 are presented by Curis Collaborator and Aurigene on November at ACR-NCI-EORTC International Conference on Molecular Targets and Cancer Therapeutics.

In particular, the immune checkpoint inhibitor is an aptamer.

Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library. The random sequence library is obtainable by combinatorial chemical synthesis of DNA. In this library, each member is a linear oligomer, eventually chemically modified, of a unique sequence.

Typically, the aptamers are directed against A2AR, B7-H3, B7-H4, BTLA, CTLA-4, CD277, IDO, KIR, PD-1, LAG-3, TIM-3 or VISTA.

In particular, aptamers are DNA aptamers such as described in Prodeus et al 2015. A major disadvantage of aptamers as therapeutic entities is their poor pharmacokinetic profiles, as these short DNA strands are rapidly removed from circulation due to renal filtration. Thus, aptamers according to the disclosure are conjugated to with high molecular weight polymers such as polyethylene glycol (PEG). In particular, the aptamer is an anti-PD-1 aptamer. Particularly, the anti-PD-1 aptamer is MP7 pegylated as described in Prodeus et al 2015.

Thus, by quantifying the percentage of CD8+ T cells co-expressing PD-1 and Tim-3 in a tumor tissue sample obtained from the subject suffering from renal cell carcinoma, physicians could determine whether the subject is eligible to a treatment with an immune check point inhibitor, thus, can adopt the treatment to said subject.

Thus, the present disclosure relates also to a method of treating renal cell carcinoma in a subject identified as a non-responder with an immune checkpoint inhibitor, comprising a step of administrating to said subject a therapeutically effective amount of a combination of immune checkpoint inhibitors.

In the context of the disclosure, the term "treatment" or "treat" as used herein, refers to both prophylactic or preventive treatment as well as curative or disease modifying treatment, including treatment of subject at risk of contracting the disease or suspected to have contracted the disease as well as subject who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

A "therapeutically effective amount" is intended for a minimal amount of active agent which is necessary to impart therapeutic benefit to a subject. For example, a "therapeutically effective amount" to a subject is such an amount which induces, ameliorates or otherwise causes an improvement in the pathological symptoms, disease progression or physiological conditions associated with or resistance to succumbing to a disorder.

As used herein, the term "combination of immune checkpoint inhibitors" refers to two inhibitors which inhibit two different immune check points concomitantly.

The inhibitor could be an antibody, synthetic or native sequence peptides, small molecules and aptamers which bind to the immune checkpoint proteins and their ligands.

In particular, the one immune checkpoint inhibitor is an anti-PD-1 and the other one is an anti-Tim-3.

In particular, the one immune checkpoint inhibitor is an anti-PD-L1 and the other one is an anti-Tim-3.

In particular, the one immune checkpoint inhibitor is an anti-PD-L2 and the other one is an anti-Tim-3.

In particular, immune check point inhibitors are antibodies. Typically, the immune checkpoint inhibitors of PD-1, PDL-1, PD-L2 and Tim-3 are antibodies as described above.

In particular, the inhibitors of PD-1 and Tim-3 are bi-specific antibodies against Tim-3 and PD-1 such as described in WO2011159877.

The immune check point inhibitors as described above may be combined with pharmaceutically acceptable excipients, and optionally sustained-release matrices, such as biodegradable polymers, to form pharmaceutical compositions. "Pharmaceutically" or "pharmaceutically acceptable" refer to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The pharmaceutical compositions of the present disclosure for oral, sublingual, subcutaneous, intramuscular, intravenous, transdermal, local or rectal administration, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Typically, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the disclosure as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms. The polypeptide (or nucleic acid encoding thereof) can be formulated into a composition in a neutral or salt form. Pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin. Sterile injectable solutions are prepared by incorporating the active polypeptides in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof. Upon formulation, solutions will be administered in a manner compatible with the dosage formulation and in such amount as is therapeutically effective. The formulations are easily administered in a variety of dosage forms, such as the type of injectable solutions described above, but drug release capsules and the like can also be employed. For parenteral administration in an aqueous solution, for example, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, sterile aqueous media which can be employed will be known to those of skill in the art in light of the present disclosure. For example, one dosage could be dissolved in 1 ml of isotonic NaCl solution and either added to 1000 ml of hypodermoclysis fluid or injected at the proposed site of infusion. Some variation in dosage will necessarily occur depending on the condition of the subject being treated. The person responsible for administration will, in any event, determine the appropriate dose for the individual subject.

The present disclosure will be further illustrated by the following figures and examples. However, these examples and figures should not be interpreted in any way as limiting the scope of the present disclosure.

### FIGURES:

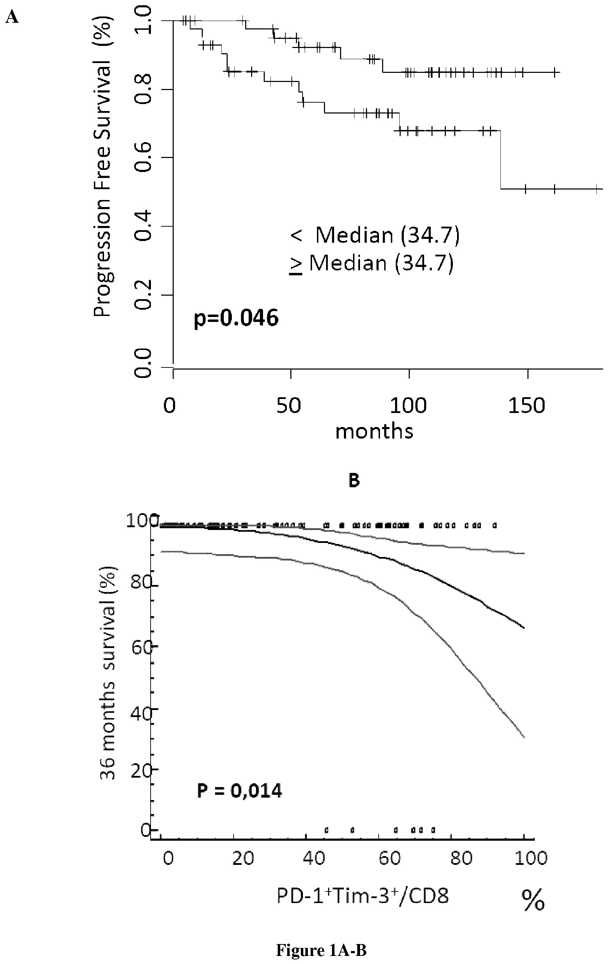

**Figure 1****: Relationships between the co-expression of PD-1 and Tim-3 on CD8+T cells and clinical outcome.** A: RCC patients were divided into two groups depending on their percent of co-expression of PD-1 and Tim-3 on CD8+T cells above or below the median (34.7). Kaplan Meier curves for the progression free survival for the two groups of patients are shown. B: The relationship between the percent of co-expression of PD-1 and Tim-3 on CD8+T cells selected as a quantitative variable and the 36- month survival is shown. The blue line corresponds to this relationship, whereas the red line represents the upper or lower limits of the 95% CI. Blue squares on the top indicate that corresponding patients are alive, while blue squares on the bottom correspond to patient death.

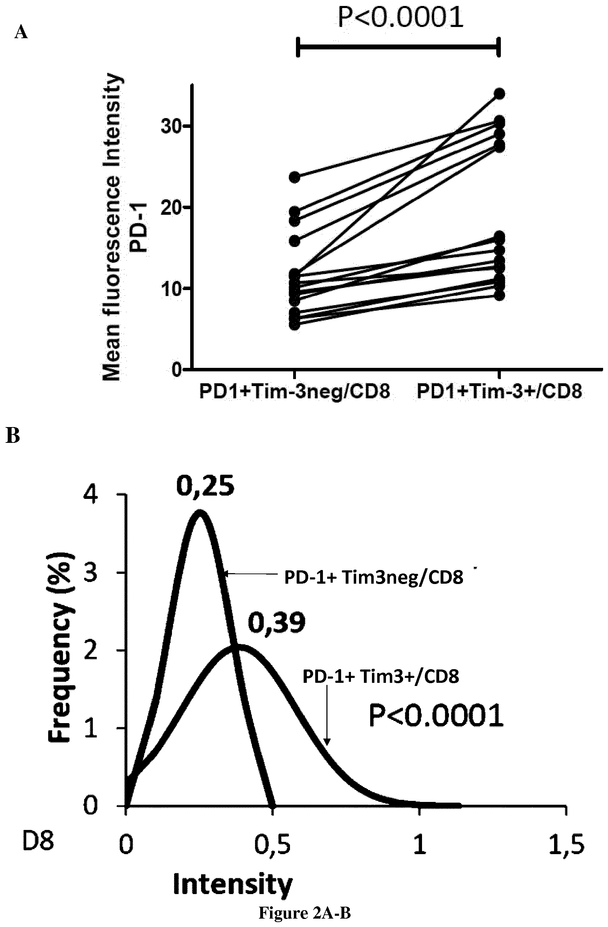

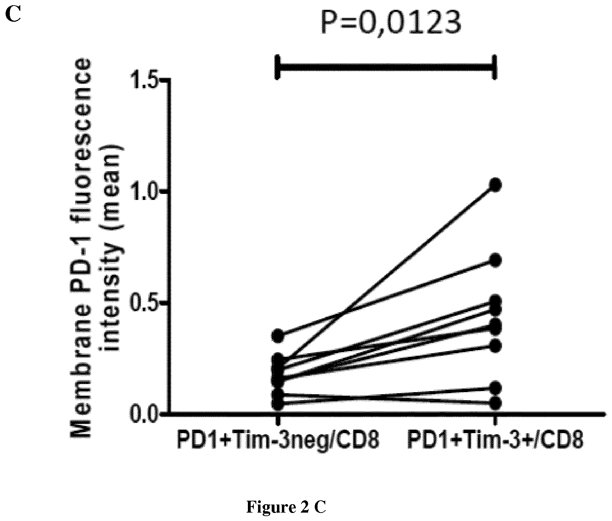

**Figure 2****: Co-expression of PD-1+Tim-3+ on CD8+T cells correlates with high levels of PD-1** A: Mean fluorescence intensity (MFI) of PD-1 measured by cytometry on PD-1+Tim-3+ and PD-1+Tim-3- cells gated on CD8+T cells for one representative RCC patient. The same analysis is shown for 16 patients selected as they coexpress PD-1 and Tim-3 at least in 10% of the total CD8+T cell population. B: Example of the intensity of PD-1 detected at the cellular levels by Immunofluorescence in situ analysis on PD-1+Tim-3+ and PD-1+Tim-3neg CD8+T cells (left) and at individual levels after integrating the various cell signal on a tissue section. Comparative analysis of the mean PD-1 intensity measure by in situ Immunofluorescence on the 2 CD8+T cell subsets (PD-1+ Tim-3- and PD-1+Tim-3+) in a series of patients for whom both tissue sections and TIL were available.

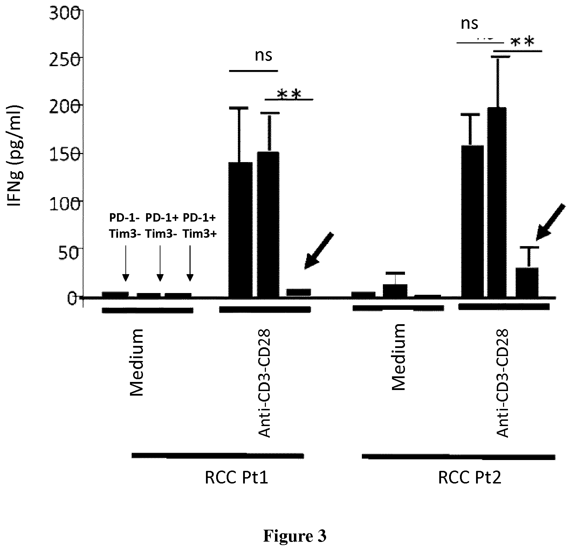

**Figure 3****: Functional analysis of TIL depending on their expression of PD-1 alone or combined with Tim-3.** Cells collected after sorting were activated or not by anti-CD3 and anti-CD28 for 24 hours and then IFN□ was measured by Elisa in the supernatant. * p < 0.01

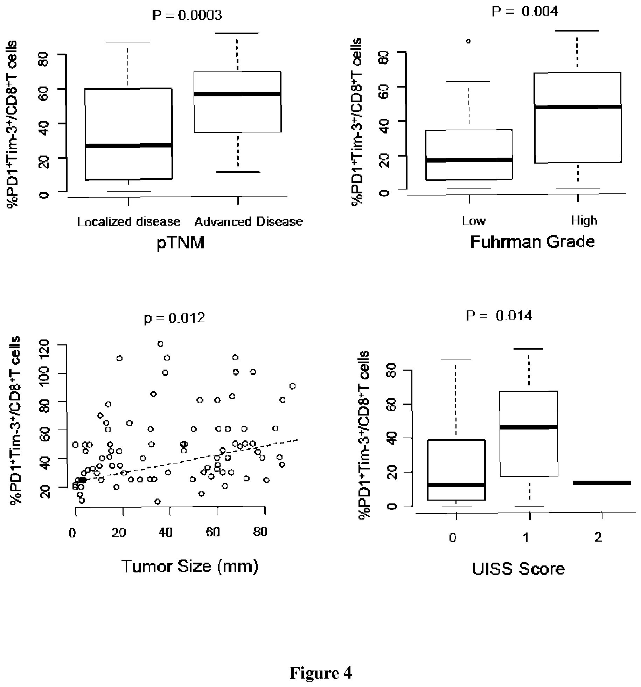

**Figure 4****: Co-expression of PD-1 and Tim-3 on CD8+T cells correlate with clinical parameters of RCC aggressivity** The percent of PD-1+Tim-3+ on CD8+T cells selected as a continuous variable and measured by in situ imunofluorescence technique was plotted against various clinical parameters defined as a binary (TNM, Fuhrman grade, UISS score) or a continuous variable (tumor size). TNM was divided in two groups : localized disease (pT1 and pT2) and advanced disease (pT3, pT4, N+ or M +). The Fuhrman grade was defined as low (grade I or II) and high (grade III or IV) and the UISS score into 3 classes (0,1,2).

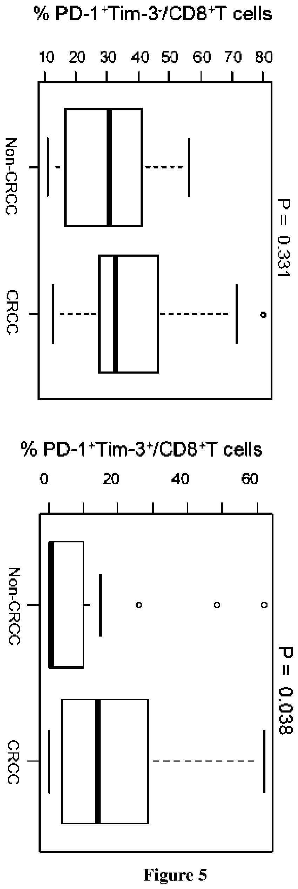

**Figure 5****: Patients who co-express PD-1 and Tim-3 on CD8+T cells more often exhibited a clear cell RCC histology** The percent of PD-1+Tim-3-and PD-1+Tim-3+ on CD8+T cells selected as a continuous variable and measured by cytometry were plotted against the histological group of RCC patients divided into two classes: Clear cell histology or non-clear cell histology (papillary, chormophobe, medullary carcinoma, oncocytoma). Box and whisker plot analysis is shown.

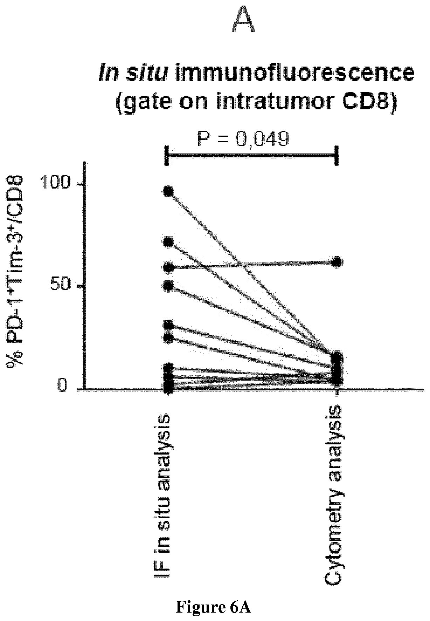

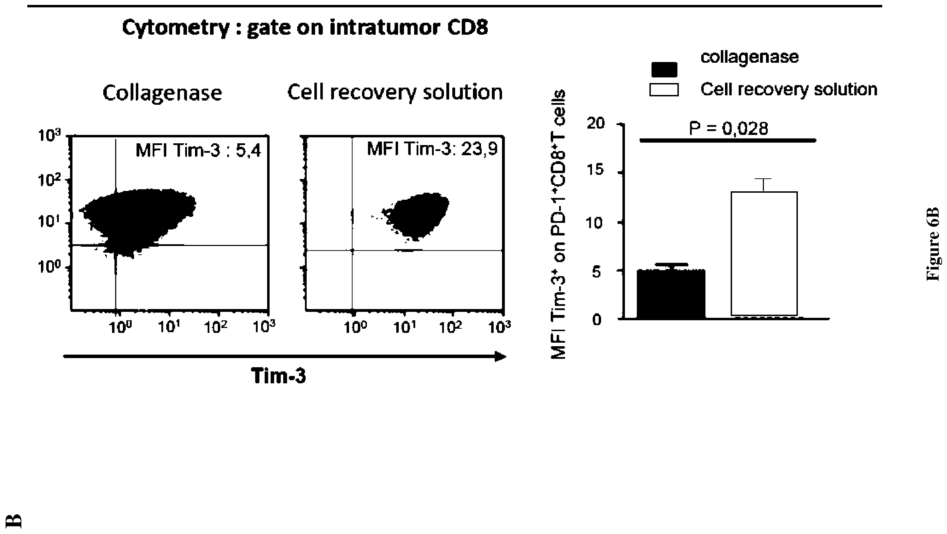

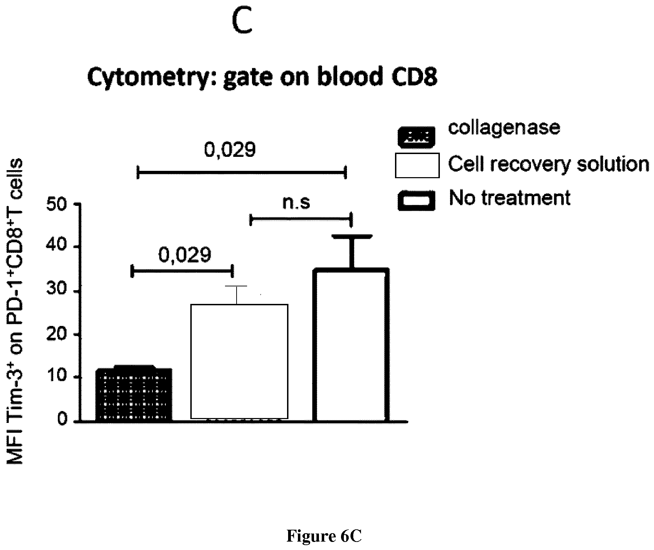

**Figure 6****: Expression of Tim-3 is decreased on PD-1+CD8+T cells after treatment with collagenase** A: The percentage of PD-1+Tim-3+/ CD8+T cells was determined by in situ immunofluorescence and cytometry: in a serie of 10 RCC patients for whom both frozen sections and fresh TIL were available. B: MFI of Tim-3 on PD-1+CD8+T cells measured by cytometry for one RCC patient whose TIL were pretreated with collagenase or cell recovery solution. Integrated data for 4 TIL derived from RCC patients. C: Four PBMC were activated by anti-CD3 and anti-CD28 for 48 hours. Then, the PBMC were treated or not with collagenase or a cell recovery solution and the MFI of Tim-3 on PD-1+CD8+T cells were measured by cytometry.

### EXAMPLE:

### Introduction

Immunotherapy based on the inhibition of checkpoint inhibitors (CTLA-4, PD-1) expressed on T cells has demonstrated their clinical efficacy in various phase 3 clinical trials in metastatic melanoma, non small cell lung carcinoma (NSCLC) and renal cell carcinoma (RCC) (1). Overall, this novel therapeutic approach leads to about 30% clinical responses in cancer patients (2). Pre-existing anti-tumor CD8+T cells seems to be required for the success of PD-1-PD-L1/2 blockade in cancer patients (3). Various arguments suggest that co-expression of inhibitory receptors (PD-1, CTLA-4, Tim-3, Lag3...) on CD8+T cells may represent a clue to explain resistance mechanisms to checkpoint inhibitors blockade. Indeed co-expression of distinct inhibitory receptors was associated with greater T cell exhaustion and resistance to the ability of anti-PD-1/PD-L1 antibodies to revigorate these dysfunctional T cells in both infections and cancer (4-7).

Up until now the co - expression of inhibitory receptors has mainly been performed on fresh tumor cells by multiparametric cytometric analysis which precludes the determination of the prognostic significance of this parameter on large cohort of patients or in retrospective studies. To overcome this hurdle, we have developed multiparametric in situ immunofluorescence analysis with multispectral imaging. The use of software for computing the pure spectrum of a fluorophore from a mixed emission signals combined with automated image analysis avoids the usual risk of overlapping signals from various fluorophores and the variation of manual counting between different operators. In human, RCC represent a good model to analyze the clinical significance of this coexpression, as some previous studies already detected inhibitory receptors (PD - 1, Lag3, PDL1, PD - L2) endowed with bad prognostic value in this cancer (8, 9). We focus on the expression of PD - 1 and Tim - 3 on CD8+T cells, as in murine acute myelogenous leukemia, human melanoma and NSCLC the co - expression of PD - 1 with Tim - 3 has been shown to correlate with T cell dysfunction (4, 10, 11). Secondary to VHL gene inactivation in most RCC with clear cell histology, there is an overexpression of VEGF explaining that RCC is a highly vascular cancer. We recently showed that VEGF induced the expression of PD - 1 and Tim - 3 on CD8+T cells (12). Lastly, TCGA data base reported a high expression of Tim - 3 in kidney renal clear cell carcinoma (13), but since Tim - 3 could also be expressed by many cells including tumor, myeloid and endothelial cells, only multiparametric in situ immunofluorescence analysis will permit to define its role and clinical significance, when expressed on CD8+T cells. The aim of this study was to evaluate the biological significance and clinical impact of the co - expression of Tim - 3 on intratumoral PD - 1+CD8+T cells in RCC patients.

### Material & methods

### Patient cohorts

Two independent cohorts of RCC patients who underwent a nephrectomy at the Urology department of European Georges Pompidou Hospital were included in this study. One of them was a prospective cohort of 42 patients enrolled between February 2012 and November 2015. All histological cancer types were included in this cohort except kystic lesions.

A second independent cohort of 87 patients who underwent surgery for a renal carcinoma between April 1999 and June 2005 was retrospectively selected from the biobank of Necker hospital for multiparametric immunofluorescence in situ analysis. These samples were directly frozen after nephrectomy and histologic assessment and only included clear cell carcinoma. Only non-treated patients harboring a clear renal cell carcinoma of were included in the two cohorts. Patient characteristics of the two cohorts are reported in Table S1 and S2. This research was conductd protocol was approved by the local ethics committee (CPP Ile de France n°2012-05-04)

### Immunophenotyping by cytometry analysis

Immunofluorescence staining and flow cytometry analysis of TILs were conducted as previously described (14). Briefly, after dissociation of biopsies by DNAse I (30 IU/mL, Roche) and Collagenase D (1mg/mL, Roche) for 60 min, cells were stained with a fixable viability Dye FVS 520 (eBioscience, Paris 75006, France), BV510 labeled anti-CD3 (BD Biosciences, Pont de Claix 38801, France), PE labeled anti-CD8 (BD Biosciences), BV421 labeled anti-PD-1 (BD Biosciences) and APC labeled anti-Tim-3 (Biolegend/Ozyme Saint Quentin Yvelines 78053 France). For the analysis, cells were gated on viable singulet positive CD3+T cells. Isotype control antibodies were included in each experiment. Detailed description of the antibodies used for the cytometry analysis are described in table S3.

### In situ TILs immunofluorescence staining

Tissue samples obtained at the day of nephrectomy were frozen and stored at -80°C. The quality of the sample was checked by an H&E stained section. Frozen specimens were sectioned at 4 to 6 µm with a cryostat, placed on slids, air dried and fixed for 5 minutes with 100% acetone. Before incubation with antibodies, the slides were pretreated with avidin/biotin blocker (DAKO) for 10 minutes and Fc receptors were blocked with Donkey serum (DAKO) 5% in TBS for 30 minutes. Staining for CD8, PD-1 and Tim3 was performed using non labeled primary antibodies followed by fluorophore labeled secondary antibodies. The antibodies used for the various immunofluorescence stainings are described in the table S3. Isotype matched antibodies were used as negative controls. In each case, we checked that the secondary antibodies did not cross react with an unrelated primary antibodies used in the combination. Nuclei were highlighted using DAPI mounting medium.

### Fluorescence analysis and automatized cell count

Slides of stained renal sections were read with an automatized microscope VectraR. This Perkin ElmerR technology allows measurement of morphometric and fluorescence characteristics in the different cell compartments (membrane/ cytoplasm/ nuclei). Coupled with an Inform software the system allows multiplex staining protocol. As recommended for the multiplex analysis, single-stained (Cyanine 5 or Cyanine 3 or Alexa Fluor 488) and non-stained slides were analyzed in Inform in order to integrate the corresponding spectrums in a fluo library.

For each slide (1 patient), image acquisition and subsequent count were done on at least 5 fields. Stained slides were visually examined by a pathologist before the analysis. The analysis of immunofluorescence labelling of the cells was performed using the Inform software. Briefly, for cell recognition, a "cell segmentation" was done based on the DAPI staining and the size. Then a sampling of 1 (under 5) image per patient was carefully examined for the phenotyping step (refer to Figure 1). Cells mono-stained for CD8 (blue dot) or co-stained for PD1 and CD8 (red dot) or PD-1, Tim-3 and CD8 (green dot) were manually identified until the automatized recognition by the Inform software was concordant with visual count (error <5%). Each images of phenotyping were checked after the software analysis.

### Cell sorting and T cell activation

Fresh tumor infiltrating lymphocytes obtained after DNAse/collagenase digestion were stained with anti-CD3, anti-CD8 , anti-PD-1 and anti-Tim-3 and were sorted into three populations PD-1+Tim-3+CD8+, PD-1+Tim-3-CD8+, PD1-Tim-3-CD8+ using a FACS-ARIA sorter (BD Biosciences). Recovered T cell were incubated for 24 hours with medium or stimulated with an anti-CD3-anti-CD28 T cell activation kit (Miltenyi). IFN□ was measured by Elisa (Diaclone) in the supernatants collected 24 hours after T cell activation or not.

**Table 1: Correlation between the expression of PD - 1 alone or combined with Tim - 3 on CD8+T cells and clinical prognostic parameters of RCC patients. The percent of PD - 1+, PD - 1+Tim - 3+ or PD - 1+Tim - 3 - on CD8+T cells selected as a continuous variable measured by either in situ imunofluorescence technique (IF) or cytometry (Cytm) was correlated against various clinical parameters defined as a binary (TNM, Fuhrman grade, UISS score) or a continuous variable (tumor size). TNM was divided in two groups : localized disease (pT1 and pT2) and advanced disease (pT3, pT4, N+ or M +). The Fuhrman grade was defined as low (grade I or II) and high (grade III or IV) and the UISS score into 3 classes (0,1, 2). The p values for significant correlation are in bold.**

| | *%PD*-*1*/*CD8* (of) | *%PD-1*/*CD8* (Cytm) | *%PD1⁺Tim-3⁺*/*CD8 (JF)* | %PD1⁺Tim-3⁺/CD8 (Cytm) | *%PD-1⁺Tim-3⁻*/*CD8 (JF)* | % PD-1⁺Tim-3⁻/CD8 (Cytm) |
|---|---|---|---|---|---|---|
| TNM | 0,04 | 0.28 | 0.003 | 0.047 | *0,22* | 0.77 |
| Furhman Grade | 0,01 | 0.25 | 0.004 | 0-56 | *0,74* | 0.33 |
| Tumor Size (mm) | 0,08 | 0.22 | 0.01 | 0.02 | 0,37 | 0.39 |
| UISS Score | 0.01 | 0.01 | 0,01 | 0.049 | *0,63* | 02 |

**Table S1: Baseline clinical characteristics of primary clear cell RCC patient selected for multiparametric immunofluorescence insitu analysis.**

| **Characteristics** | **Number of patients (%)** |
|---|---|
| **Sex** | |
| • Male | 60 (69%) |
| • Female | 27 (31%) |
| **Median Age** (Years) | 76 (46-101) |

| **pTNM** | |
|---|---|
| pT1-T2 (localized disease) | |
| - pT1 | 60 (69%) |
| - pT2 | 3 (3%) |
| • pT3-T4-N+M+ (advanced disease) | |
| - pT3 | 24 (28%) |
| - pT4 | 0 |
| - N+ | 0 |
| - M+ | 0 |
| **Sarcomatoid component** | 11 (13%) |
| **Tumor Size (major Axis (mm))** | Median 4, mean 4.7 (10-120) |

| **Fuhrman Grade** | |
|---|---|
| • Grade I and II (low) | |
| - Grade I | 0 (0%) |
| - Grade II | 25 (29%) |
| • Grade III and IV (high) | |
| - Grade III | 41 (47%) |
| - Grade IV | 21 (24%) |

| **UISS Score** | |
|---|---|
| • Low | 22 (25.3%) |
| •Intermediate | 62 (71.3%) |
| • High | 3 (3.4%) |

**Table S2: Baseline clinical characteristics of primary RCC patient with analysis of fresh tumor TIL. The Fuhrman grade could only be assessed for clear cell and papillary histology type. Missing data for 1 patients for Fuhrman grade and 3 for UISS score.**

| **Characteristics** | **Number of patients (%)** |
|---|---|
| **Sex** | |
| • **Male** | **27(64-3)** |
| • **Female** | **15 (35.7)** |
| **Median Age (Years)** | **56 (2B-B1)** |

| **pTNM** | |
|---|---|
| • **pT1-T2 (localized disease)** | **24 (57.15)** |
| - **pT1** | **17 (40.5)** |
| - **pT2** | **7 (16.65)** |
| • **pT3-T4-N⁺M⁺ (advanced disease)** | **18 (42.85)** |
| - **pT3** | **18** |
| - **pT4** | **0** |
| - **N+** | **2 (1)** |
| - **M+** | **1 (0.05)** |

| **Histology Type** | |
|---|---|
| • **Clear Cell** | **27 (64,3)** |
| • **Papillary** | **7 (16.7)** |
| • **Chromophobe** | **6 (14-2)** |
| • **Others (medullary carci noma and oncocytoma)** | **2 (4-8)** |
| **Saroomatoid component** | **1 (2-4)** |
| **Tumor Size (major Axis [mm])** | **60 (20-110)** |

| **Fuhrman Grade** | |
|---|---|
| • **Grade** I **and II (low)** | **11 (33.33)** |
| - **Grade I** | **0** |
| - **Grade II** | **11 (33.33)** |
| • **Grade III and IV (high)** | **22 (66.66)** |
| - **Grade III** | **15 (45-45)** |
| - **Grade IV** | **7(21-2)** |

| **UISS Score** | |
|---|---|
| • **Low** | 6 |
| • **Intermediate** | 27 |
| • High | 0 |

**Table S3: List of antibodies used for in situ immunofluorescence staining and cytometry**

| | Primary antibodies | Secondary antibodies | Revelation |
|---|---|---|---|
| Cytometry | | | |
| CD3-CD8-PD-1-Tim-3 | BV510 conjugated anti-CD3 (Clone UCHT1)(BD Biosciences) | | |
| | - PE conjugated Anti-CD8 (Clone- RPA-T8) | | |
| | - (BD Biosciences) | | |
| | - BV421 conjugated anti-PD-1 Clone MIH4) (BD Biosciences) | | |
| | APC conjugated anti-Tim-3 (Clone F38-2E2)(Biolegend) | | |

| **In situ Immunofluorescence analysis** | | | |
|---|---|---|---|
| CDB-PD-1-Tim-3 | Rabbit anti-CDS (Clone P17-V(Novus) | Cyan 5 conjugated donkey anti-rabbit (Jackson Immunoresearch) Biotynalted F(ab'2) donkey antimouse IgG (Jackson Immunoresearch Alexa Fluor R 488 conjugated donkey anti-goat IgG (Abcam) | Cy™3 labeled streptavidin (Amesham) |
| | Mouse anti-PD-1 (Clone NAT)(Abcam) | | |
| | Goat anti-Tim-3 (R&D) | | |

**Table S4: Correlation between the total number (Nb) of CD8+T cells and those expressing Tim-3 and/or PD-1.**

| | Nb CD8 ⁺T cell | Nb PD-1⁺CD8 ⁺T cell | Nb PD-1⁺Tim-3⁺ CDB⁺T cell | Nb PD1⁺Tim3TCD8⁺T cell |
|---|---|---|---|---|
| Furhman | 0,029 | 0,0055 | 0,0024 | D,1 |
| UISS | D,17 | 0,0229 | 0,012 | 0,17 |

### Results

### 1) Detection and characterization of CD8+T cells co-expressing or not PD-1 and Tim-3 by automated in situ immunofluorescence spectral imaging.

To characterize the CD8+T cells infiltrating renal cell carcinoma and expressing PD-1 and Tim-3, we set up a multifluorescence in situ technique with automated counting. We first showed that about half of CD8+T cell express PD-1 (mean 53.9%; SE : 30.49%). This population could be divided into two groups: i) one corresponding to double positive PD-1+Tim-3+ within CD8+T cells with a mean percent of 38.16% (SE: 28.11%) i) a second population of CD8+T cells expressing PD-1 without Tim-3 (mean : 15.77% ; SE : 8.62%). Thus within tumor microenvironment, most PD-1+CD8+T cell coexpressed Tim-3, whereas Tim-3 without PD-1 was detected in less than 3% of CD8+T cells (data not shown). The mean number of total CD8+T cells, PD-1+CD8+T cells, PD-1+Tim-3+ and PD-1+Tim-3 negative CD8+T cells were 116.5 (SE : 216), 89.32 (SE : 191.8), 66.9 (SE : 143), 22.38 (SE : 57.5) respectively. As expected we also observed the expression of Tim-3 on non CD8+T cells.

### 2) Clinical significance of the co-expression of PD-1 and Tim-3 on CD8+T cells in situ.

Various criteria (TNM, Fuhrman grade, size of the tumor, UISS score) have been proposed to define the prognostic value of primary RCC. The percent or the number of intratumor CD8+T cells expressing PD-1 without Tim-3 did not correlate with any criteria of aggressivity defined above (Table 1 and Table S4). In contrast, a positive relationship was observed between the percent of intratumor CD8+T cells expressing PD-1 (i.e. Tim-3+ or Tim-3neg) or co-expressing PD-1 and Tim-3 and the TNM stage, the Fuhrman grade and the UISS score (Table 1). Coexpression of PD-1 and Tim-3 was also associated with a larger tumor size. In addition, the number of tumor infiltrating CD8+T cells expressing PD-1 or co-expressing PD-1 and Tim-3 correlated with the Fuhrman grade and the UISS score (Table S4). In line with this more pejorative phenotype, RCC patients whose CD8+T cells co-express PD-1 and Tim-3 above the median (34.7) are more likely to relapse (p = 0.046 ; HR 2.9; 95% confidence interval (CI): 1.02-8.21)(Fig 1A). A correlation was also shown between the percent of CD8+T cells coexpressing PD-1 and Tim-3 and 36 months overall survival (Fig1B). This same group of patients had also a trend toward a poorer overall survival when median was selected as a cut-off (p = 0.079; HR 2.16; 95% CI : 0.91-5.1). When all other CD8 subsets were set up as a continuous variable or binary variable defined by the median, no significant statistical correlation were found with progression free survival (PFS) or overall survival (OS). Only the percent of the co-expression of PD-1 and Tim-3 on CD8+T cells had an impact on the clinical outcome of patients. In a multi-variate model including the percent of the double positive PD-1+Tim+3+CD8+T cells, the TNM classification and the size of the tumor, only the size of the tumor remains significantly associated with the PFS (p = 0.0099).

### 3) Assessment of the co-expression of PD-1 and Tim-3 on CD8+T cells by cytometry and its clinical significance.

To validate these results, we measure the expression of PD - 1 and Tim - 3 on CD8+T cell by cytometry in a series of 42 fresh tumors derived from RCC patients. As previously observed with the multiparametric immunofluorescence in situ technique, about half of CD8+T cells express PD - 1 (mean 50,8 + 20.76) and none CD8+T cells express Tim - 3 without PD - 1. In the whole population, 15% of CD8+T cells co - express PD - 1 and Tim - 3 and this percent increases to 17.42% in the restricted CRCC patients. Although, the two series of patients were independent, we were surprised that the percent of PD - 1 expression on CD8+T cells fit with the two techniques but not those of Tim - 3 expression. We first confirmed that this difference was not secondary to the independent series of patients tested. Indeed, Fig 6A showed that for the same group of 10 RCC patients for whom both TIL and frozen section were available, the percent of PD - 1+Tim - 3+ on CD8+T cells was higher when detected by in situ immunofluorescence technique than by cytometry (p = 0.049). We further explain this discordance by showing that TIL treated by collagenase have a decrease in the expression of Tim - 3 compared to the use of mechanical method of dissociation (p = 0.028) (Fig 6B). Unfortunately, this mechanical method has a low yield of recovered cells compared to the use of collagenase (data not shown). The expression of Tim - 3 on activated PBMC were also diminished, when they were treated by collagenase compared to the mechanical dissociation exposure or the absence of treatment (p = 0.029) (Fig 6). Interestingly, collagenase did not affect the PD - 1 expression of CD8+T cells explaining the concordance for this marker between these two techniques. These results support the clinical value of in situ immunofluorescence multiparametric technique to avoid this kind of bias. Regarding the clinical significance of results obtained by cytometry, we confirmed that the percent of CD8+T cells expressing PD - 1 without Tim - 3 by cytometry analysis did not correlate with any prognostic criteria (TNM, Fuhrman grade, size of the tumor, UISS score), while patients whose CD8+T cells co - express PD - 1 and Tim - 3 had a more advanced TNM stage (p = 0.021), a tumor with a larger size (p = 0.021) and a higher UISS score (p = 0.049). Interestingly, the co-expression of Tim - 3 and PD - 1 on CD8+T cells was higher in the group patients with a CRCC histology which is considered as a more aggressive cancer than the chromophobe or tubulo - papillary or oncocytoma subgroups (Fig 5). Since the cytometry study was a prospective analysis, the follow up was not long enough to assess the PFS and the OS of this group of patients. So except for the absolute percent of the co - expression of PD - 1 and Tim - 3 on CD8+T cells, the other parameters measured and their clinical significance were very similar between the two series.

### 4) Phenotypic and functional characterization of the population of CD8+T cells co-expressing PD-1 and Tim-3

Since it has been shown that the levels of PD-1 on T cells correlated with the exhaustion of T cells defined by multiple expression of inhibitory receptors (15), we quantitated in a series of 16 patients the expression of PD-1 on CD8+T cells co-expressing or not Tim-3 by cytometry. Patients were selected based on the coexpression of PD - 1 and Tim - 3 at least of 10% of the total CD8+T cell population. In the patient shown in Fig 2A, we observed an increased expression of the mean fluorescence intensity (MFI) of PD - 1, when it co - expressed Tim - 3 (MFI : 10.4) compared to its expression alone (5.88). For the whole population, the MFI of PD - 1 on CD8+T cells co - expressing Tim - 3 (mean 19.13 + 8.8) was also higher than the MFI observed on CD8+T cells not expressing Tim - 3 (mean 11.58 + 5.2) (p = 0.0063) (Fig 2A). This result was confirmed when in situ immunofluorescence analysis was performed and the intensity levels of PD - 1 compared, when it was co - expressed or not with Tim - 3 on CD8+T cells. Indeed, in the absence of Tim - 3, the mean of PD - 1 fluorescence intensity was measured at 0.31 (+ 0.15) and it increased to 0.489 (+ 0.19), when it was co - expressed with Tim - 3 (Fig 2B) (p = 0.0418). Thus by using two different techniques we demonstrated that the co - expression of PD - 1 and Tim - 3 was associated with an increased levels of PD - 1 on CD8+T cells. To determine the putative difference in term of functionality between the PD - 1+Tim - 3+ CD8+T cells and the PD - 1+Tim - 3negCD8+T cell population, we selected two patients which coexpress PD - 1 and Tim - 3 and sorted the three following populations: PD - InegTim - 3negCD8+T cells, PD - 1+Tim - 3negCD8+T cells and PD - 1+Tim - 3+CD8+T cells (Fig 3). After a stimulation with anti - CD3 and anti - CD28 mAb we showed that the PD1 - Tim3 - and the PD1+Tim3 - population secrete large amount of IFN in their supernatants with no significant difference between these two subpopulations. In contrast, the production of IFN□ was significantly decreased in the CD8+T cells co - expressing PD1 and Tim - 3 compared to the other two populations (Fig 3).

### Discussion

Using a novel spectral multi-immunofluorescence in situ imaging technology, we showed that the clinical significance of PD-1 on CD8+T cells differs, whether it was co-expressed or not with Tim-3. Indeed, we showed that renal cell cancer patients infiltrated with CD8 cells that co-expressed PD-1 and Tim-3 had a more aggressive phenotype defined by high Fuhrman grade and tumor of larger size and advanced TNM and UISS score. In addition, this group of patients also exhibited a lower PFS and reduced overall survival at 36 months. We confirmed this aggressive phenotype by cytometry analysis, as patients whose CD8+T cells co-expressed PD-1 and Tim-3 had a more advanced TNM stage and UISS score and a larger tumor size. These data may explain some controversies in the literature about the prognostic value of PD-1 (8, 15-18) and emphasized about the critical role of the combined expression of coinhibitory receptors especially Tim-3 in the clinical significance of PD-1. This complex interpretation of the clinical value of PD-1 parallels its multiple biological significance. Indeed, PD-1 is both an activation marker and a hallmark of exhausted T cells. However, PD- 1 also preserves CD8+T cells from overstimulation and the risk of accumulation of terminally differentiated exhausted CD8+T cells (19). Although Tim-3 is also induced after activation (20), its co-expression with PD-1 in the tumor microenvironment may represent a switch leading to compromised functionality of T cells (4, 20, 21). We showed that the CD8+T cell population co-expressing PD-1 and Tim-3 presented all the features of an exhausted T cell population, as they respond poorly to T cell stimulation. In addition, high levels of PD-1 expressed at their membrane are considered as a hallmark of a particularly dysfunctional T cell (11, 14). Interestingly both in vitro and in vivo, Tim - 3 - Tim - 3 ligand blockade in combination with the inhibition of the PD - 1 - PD - L1 pathway synergized to restore T cell function resulting in the control of chronic infection and inhibition of tumor growth (4, 5, 22, 23). Besides activation, Th1 cytokines may favor this co - expression of PD - 1 and Tim - 3, as type I and II IFN regulated PD - 1 and IL - 12 enhanced the expression of Tim - 3 (20, 24). Tumor associated M2 macrophages also regulated the expression of Tim - 3 on T cells derived from RCC (25). We recently showed that VEGF also enhanced the expression of PD - 1 and Tim - 3 after activation (12). Tim - 3 could also be expressed on non T cells such as myeloid cells conferring to these cells an impaired immunosurveillance (26, 27). In RCC, Tim - 3 has been shown to be expressed in macrophages and in tumor cells (28). Tim - 3 promoted ccRCC invasion and rendered these cells more resistant to anti - angiogenic molecules (28). Intratumor Tim - 3+CD8+T cells have been correlated with histological grade and advanced tumor stage in follicular lymphoma and NSCLC respectively but with no data on their influence on the clinical outcome (11, 20). Furthermore, higher expression of Tim - 3 gene expression in kidney renal cell carcinoma was a marker for worse 5 - year survival (13). In contrast to cancer, in preneoplastic lesions such as usual - type vulvar intra epithelial neoplasia, the significance of Tim - 3+CD8+T cells may be less pejorative, as it was related with an absence of recurrence. However, the number of Tim - 3+CD8+T cells increased in vulvar carcinoma compared to benign lesions (29). All these data converge for the targeting of Tim - 3 in cancer alone or preferentially in combination with anti - PD - 1/PD - L1. Other checkpoint inhibitors such as Lag - 3 could be co - expressed with PD - 1 on CD8+T cells as shown in RCC and other tumors and it usually correlated with an impaired effector function of these cells (9, 30). Interestingly, in NSCLC, CD8+T cells expressing Tim - 3 are those which co - expressed the higher number of other inhibitory receptors compared to cells expressing other checkpoint inhibitors possibly making Tim - 3 as a surrogate marker of more advanced exhausted T cells (11). One limit of this study is that we did not compartimentalize our CD8+T cell population in the tumor core or in the stroma due to difficulties to combine a homogenous tumor cell marker with our set of T cell antibodies. But, renal carcinomas are not histological entities with welldefined invasive margin as observed in some other epithelial tumors (i.e colorectal cancer). As it has been shown that the prognostic value of subpopulations of T cells depends on their location in the nest of the tumor or in the periphery, it could explain some minor discrepancies between our results and report in the literature regarding the prognosis value of the number of CD8+T cells and PD - 1+T cells (8, 9, 31). It could also explain the more significant impact of the percent expression of the inhibitory receptors , PD - 1 and Tim - 3 over the number of cells expressing them, as this percent reflects an intrinsic status of the exhausted state of the intra - tumor CD8+T cells. The influence of the number of PD - 1+Tim 3+CD8+T cells may be more dependent on their ratio with the number of tumor cells. In addition, in most reports from the literature, a monoparametric immunochemistry technique for the analysis of checkpoint inhibitor expression have been employed which differed from our focus on the characterization of checkpoint inhibitors specifically on CD8+T cells considered as one of the main effectors after immunotherapy (3). We also selected the various variables either as a continuous variable or with a median cut - off, which differs from previous studies that used optimal p value (8, 9). Novel multiparametric in situ technology set up in this study and recently described by other groups (3, 32) will allow a better characterization of CD8+T cells and other immune cells at single cell levels in the tumor microenvironment to better guide the choice of immune target for immunotherapy. We showed that for some parameters such as Tim - 3, collagenase may decrease their expression when detected by cytometry which reinforces the value of our in situ multiparametric automated immunofluorescence technique to directly assess in vivo the intratumor expression of checkpoint inhibitor in untouched cells. In addition, the fact that in contrast to the PD - 1+Tim - 3neg CD8+T cell population, the double positive PD - 1+Tim - 3+CD8+T cells could not be activated in vitro with a strong stimulus suggest that it could also be difficult to revigorate them after PD - PDL - 1 blockade and thus constitutes a biomarker of resistance to immunotherapy.

### REFERENCES:

Throughout this application, various references describe the state of the art to which the present disclosure pertains.
1. Lipson EJ, et al. (2015) Antagonists of PD - 1 and PD - L1 in Cancer Treatment. Seminars in oncology 42(4):587 - 600.
2. Sharma P & Allison JP (2015) The future of immune checkpoint therapy. Science 348(6230):56 - 61.
3. Turmeh PC, et al. (2014) PD - 1 blockade induces responses by inhibiting adaptive immune resistance. Nature 515(7528):568 - 571.
4. Fourcade J, et al. (2010) Upregulation of Tim - 3 and PD - 1 expression is associated with tumor antigen - specific CD8+ T cell dysfunction in melanoma patients. J Exp Med 207(10):2175 - 2186.
5. Sakuishi K, et al. (2010) Targeting Tim - 3 and PD - 1 pathways to reverse T cell exhaustion and restore anti - tumor immunity. J Exp Med 207(10):2187 - 2194.
6. Baitsch L, Fuertes - Marraco SA, Legat A, Meyer C, & Speiser DE (2012) The three main stumbling blocks for anticancer T cells. Trends Immunol 33(7):364 - 372.
7. Wherry EJ & Kurachi M (2015) Molecular and cellular insights into T cell exhaustion. Nat Rev Immunol 15(8):486 - 499.
8. Thompson RH, Dong H, & Kwon ED (2007) Implications of B7 - H1 expression in clear cell carcinoma of the kidney for prognostication and therapy. Clin Cancer Res 13(2 Pt 2):709s 715s.
9. Giraldo NA, et al. (2015) Orchestration and Prognostic Significance of Immune Checkpoints in the Microenvironment of Primary and Metastatic Renal Cell Cancer. Clin Cancer Res 21(13):3031 - 3040.
10. Zhou Q, et al. (2011) Coexpression of Tim - 3 and PD - 1 identifies a CD8+ T-cell exhaustion phenotype in mice with disseminated acute myelogenous leukemia. Blood 117(17):4501 - 4510.
11. Thommen DS, et al. (2015) Progression of Lung Cancer Is Associated with Increased Dysfunction of T Cells Defined by Coexpression of Multiple Inhibitory Receptors. Cancer Immunol Res.
12. Voron T, et al. (2015) VEGF - A modulates expression of inhibitory checkpoints on CD8+ T cells in tumors. J Exp Med 212(2):139 - 148.
13. Zheng H, Guo X, Tian Q, Li H, & Zhu Y (2015) Distinct role of Tim - 3 in systemic lupus erythematosus and clear cell renal cell carcinoma. International journal of clinical and experimental medicine 8(5):7029 - 7038.
14. Blackburn SD, Shin H, Freeman GJ, & Wherry EJ (2008) Selective expansion of a subset of exhausted CD8 T cells by alphaPD - L1 blockade. Proc Natl Acad Sci U S A 105(39):15016 - 15021.
15. Carreras J, et al. (2009) High numbers of tumor - infiltrating programmed cell death 1 - positive regulatory lymphocytes are associated with improved overall survival in follicular lymphoma. J Clin Oncol 27(9): 1470 - 1476.
16. Badoual C, et al. (2013) PD - 1 - expressing tumor - infiltrating T cells are a favorable prognostic biomarker in HPV - associated head and neck cancer. Cancer Res 73(1): 128 - 138.
17. Muenst S, et al. (2013) The presence of programmed death 1 (PD - 1) - positive tumorinfiltrating lymphocytes is associated with poor prognosis in human breast cancer. Breast cancer research and treatment 139(3):667 - 676.
18. Mlecnik B, et al. (2010) Biomolecular network reconstruction identifies T - cell homing factors associated with survival in colorectal cancer. Gastroenterology 138(4):1429 - 1440.
19. Odorizzi PM, Pauken KE, Paley MA, Sharpe A, & Wherry EJ (2015) Genetic absence of PD - 1 promotes accumulation of terminally differentiated exhausted CD8+ T cells. J Exp Med 212(7): 1125 - 1137.
20. Yang ZZ, et al. (2012) IL - 12 upregulates TIM - 3 expression and induces T cell exhaustion in patients with follicular B cell non - Hodgkin lymphoma. The Journal of clinical investigation 122(4): 1271 - 1282.
21. Severson JJ, et al. (2015) PD - 1+Tim - 3+ CD8+ T Lymphocytes Display Varied Degrees of Functional Exhaustion in Patients with Regionally Metastatic Differentiated Thyroid Cancer. Cancer Immunol Res 3(6):620 - 630.
22. Takamura S, et al. (2010) Premature terminal exhaustion of Friend virus - specific effector CD8+ T cells by rapid induction of multiple inhibitory receptors. J Immunol 184(9):4696 - 4707.
23. Jin HT, et al. (2010) Cooperation of Tim - 3 and PD - 1 in CD8 T - cell exhaustion during chronic viral infection. Proc Natl Acad Sci U S A 107(33): 14733 - 14738.
24. Terawaki S, et al. (2011) IFN - alpha directly promotes programmed cell death - 1 transcription and limits the duration of T cell - mediated immunity. J Immunol 186(5):2772 - 2779.
25. Dannenmann SR, et al. (2013) Tumor - associated macrophages subvert T - cell function and correlate with reduced survival in clear cell renal cell carcinoma. Oncoimmunology 2(3):e23562.
26. Tang D & Lotze MT (2012) Tumor immunity times out: TIM - 3 and HMGB1. Nature immunology 13(9):808 - 810.
27. Chiba S, et al. (2012) Tumor - infiltrating DCs suppress nucleic acid - mediated innate immune responses through interactions between the receptor TIM - 3 and the alarmin HMGB1. Nature immunology 13(9):832 - 842.
28. Komohara Y, et al. (2015) The Coordinated Actions of TIM - 3 on Cancer and Myeloid Cells in the Regulation of Tumorigenicity and Clinical Prognosis in Clear Cell Renal Cell Carcinomas. Cancer Immunol Res 3(9):999 - 1007.
29. van Esch EM, et al. (2015) Expression of coinhibitory receptors on T cells in the microenvironment of usual vulvar intraepithelial neoplasia is related to proinflammatory effector T cells and an increased recurrence - free survival. Int J Cancer 136(4):E95 - 106.
30. Matsuzaki J, et al. (2010) Tumor - infiltrating NY - ESO - 1 - specific CD8+ T cells are negatively regulated by LAG - 3 and PD - 1 in human ovarian cancer. Proc Natl Acad Sci U SA 107(17):7875 - 7880.
31. Nakano O, et al. (2001) Proliferative activity of intratumoral CD8(+) T - lymphocytes as a prognostic factor in human renal cell carcinoma: clinicopathologic demonstration of antitumor immunity. Cancer Res 61(13):5132 - 5136.
32. Schalper KA, et al. (2015) Objective measurement and clinical significance of TILs in non - small cell lung cancer. J Natl Cancer Inst 107(3). 33. R Core Team (2015) R: A language and environment for statistical computing.

## Claims

1. A method for predicting the survival time of a subject suffering from renal cell carcinoma comprising the steps of: i) quantifying the percent of CD8+ T cells co-expressing PD-1 and Tim-3 in a tumor tissue sample obtained from the subject, ii) comparing the percent quantified at step i) with its corresponding predetermined reference value and iii) concluding that the subject will have a short survival time when the percent of CD8+ T cells co-expressing PD-1 and Tim-3 is higher than its corresponding predetermined reference value or concluding that the subject will have a long survival time when the percent of CD8+ T cells co-expressing PD-1 and Tim-3 is lower than its corresponding predetermined reference value.

2. The method of claim 1 wherein, the quantification of percent of CD8+ T cells co-expressing PD-1 and Tim-3 is determined by Immunohistochemistry (IHC).

3. The method of claim 1 wherein, the quantification of percent of CD8+ T cells co-expressing PD-1 and Tim-3 is determined by an automatized microscope.

4. A method for determining whether a subject suffering from a renal cell carcinoma will achieve a response with an immune-checkpoint inhibitor comprising the steps of i) quantifying the percent of CD8+ T cells co-expressing PD-1 and Tim-3 in a tumor tissue sample obtained from the subject treated with an immune-checkpoint inhibitor, ii) comparing the percent CD8+ T cells co-expressing PD-1 and Tim-3 quantified at step i) with its corresponding predetermined reference values and iii) concluding that the subject will not respond to the treatment when the percent of CD8+ T cells co-expressing PD-1 and Tim-3 is higher than its corresponding predetermined reference value or concluding that the subject will respond to the treatment when the percent of CD8+ T cells co-expressing PD-1 and Tim-3 is lower than its corresponding predetermined reference value.

5. The method of claim 4, wherein the immune checkpoint inhibitor is an antibody.

6. The method of claim 4, wherein the immune checkpoint inhibitor is a monoclonal antibody.

7. The method of claim 4, wherein the immune checkpoint inhibitor is an anti-PD-1 antibody.

8. The method of claim 4, wherein the immune checkpoint inhibitor is an anti-PD-L1 antibody.

9. The method of claim 4, wherein the immune checkpoint inhibitor is an anti-PD-L2 antibody.

10. The method of claim 4, wherein the immune checkpoint inhibitor is an anti-Tim-3 antibody.

11. The method of claim 4, wherein the immune checkpoint inhibitor is a small organic molecule.

12. The method of claim 4, wherein the immune checkpoint inhibitor is an aptamer.

## Patentansprüche

1. Verfahren zum Vorhersagen der Überlebenszeit einer Person, die an einem Nierenzellkarzinom leidet, umfassend die Schritte: i) Quantifizieren der Prozent von CD8+-T-Zellen, die PD-1 und Tim-3 in einer von der Person erhaltenen Tumorgewebeprobe coexprimieren, ii) Vergleichen der bei Schritt i) quantifizierten Prozent mit ihrem entsprechenden vorbestimmten Bezugswert und iii) Folgern, dass die Person eine kurze Überlebenszeit haben wird, wenn die Prozent von CD8+-T-Zellen, die PD-1 und Tim-3 coexprimieren, höher als ihr entsprechender vorbestimmter Bezugswert sind, oder Folgern, dass die Person eine lange Überlebenszeit haben wird, wenn die Prozent von CD8+-T-Zellen, die PD-1 und Tim-3 coexprimieren, niedriger als ihr entsprechender vorbestimmter Bezugswert sind.

2. Verfahren nach Anspruch 1, wobei die Quantifizierung von Prozent von CD8+-T-Zellen, die PD-1 und Tim-3 coexprimieren, durch Immunhistochemie (IHC) bestimmt wird.

3. Verfahren nach Anspruch 1, wobei die Quantifizierung von Prozent von CD8+-T-Zellen, die PD-1 und Tim-3 coexprimieren, durch ein automatisiertes Mikroskop bestimmt wird.

4. Verfahren zum Bestimmen, ob eine Person, die an einem Nierenzellkarzinom leidet, mit einem Immuncheckpoint-Inhibitor eine Reaktion erreichen wird, umfassend die Schritte i) Quantifizieren der Prozent von CD8+-T-Zellen, die PD-1 und Tim-3 in einer von der mit einem Immuncheckpoint-Inhibitor behandelten Person erhaltenen Tumorgewebeprobe coexprimieren, ii) Vergleichen der bei Schritt i) quantifizierten Prozent von CD8+-T-Zellen, die PD-1 und Tim-3 coexprimieren, mit ihren entsprechenden vorbestimmten Bezugswerten und iii) Folgern, dass die Person nicht auf die Behandlung reagieren wird, wenn die Prozent von CD8+-T-Zellen, die PD-1 und Tim-3 coexprimieren, höher als ihr entsprechender vorbestimmter Bezugswert sind, oder Folgern, dass die Person auf die Behandlung reagieren wird, wenn die Prozent von CD8+-T-Zellen, die PD-1 und Tim-3 coexprimieren, niedriger als ihr entsprechender vorbestimmter Bezugswert sind.

5. Verfahren nach Anspruch 4, wobei der Immuncheckpoint-Inhibitor ein Antikörper ist.

6. Verfahren nach Anspruch 4, wobei der Immuncheckpoint-Inhibitor ein monoklonaler Antikörper ist.

7. Verfahren nach Anspruch 4, wobei der Immuncheckpoint-Inhibitor ein Anti-PD-1-Antikörper ist.

8. Verfahren nach Anspruch 4, wobei der Immuncheckpoint-Inhibitor ein Anti-PD-Ll-Antikörper ist.

9. Verfahren nach Anspruch 4, wobei der Immuncheckpoint-Inhibitor ein Anti-PD-L2-Antikörper ist.

10. Verfahren nach Anspruch 4, wobei der Immuncheckpoint-Inhibitor ein Anti-Tim-3-Antikörper ist.

11. Verfahren nach Anspruch 4, wobei der Immuncheckpoint-Inhibitor ein kleines organisches Molekül ist.

12. Verfahren nach Anspruch 4, wobei der Immuncheckpoint-Inhibitor ein Aptamer ist.

## Revendications

1. Procédé de prédiction de la durée de survie d'un sujet souffrant d'un carcinome des cellules rénales comprenant les étapes suivantes : i) la quantification du pourcentage de cellules T CD8+ co-exprimant PD-1 et Tim-3 dans un échantillon de tissu tumoral obtenu à partir du sujet, ii) la comparaison du pourcentage quantifié à l'étape i) avec sa valeur de référence prédéterminée correspondante et iii) conclure que le sujet présentera une courte durée de survie quand le pourcentage de cellules T CD8+ co-exprimant PD-1 et Tim-3 est supérieur à sa valeur de référence prédéterminée correspondante ou conclure que le sujet présentera une longue durée de survie quand le pourcentage de cellules T CD8+ co-exprimant PD-1 et Tim-3 est inférieur à sa valeur de référence prédéterminée correspondante.

2. Procédé selon la revendication 1 dans lequel, la quantification du pourcentage de cellules T CD8+ co-exprimant PD-1 et Tim-3 est déterminée par immunohistochimie (IHC).

3. Procédé selon la revendication 1 dans lequel, la quantification du pourcentage de cellules T CD8+ co-exprimant PD-1 et Tim-3 est déterminée par un microscope automatisé.

4. Procédé pour déterminer si un sujet souffrant d'un carcinome des cellules rénales obtiendra une réponse avec un inhibiteur de point de contrôle immunitaire comprenant les étapes suivantes i) la quantification du pourcentage de cellules T CD8+ co-exprimant PD-1 et Tim-3 dans un échantillon de tissu tumoral obtenu à partir du sujet traité avec un inhibiteur de point de contrôle immunitaire, ii) la comparaison du pourcentage de cellules T CD8+ co-exprimant PD-1 et Tim-3 quantifié à l'étape i) avec ses valeurs de référence prédéterminées correspondantes et iii) conclure que le sujet ne répondra pas au traitement quand le pourcentage de cellules T CD8+ co-exprimant PD-1 et Tim-3 est supérieur à sa valeur de référence prédéterminée correspondante ou conclure que le sujet répondra au traitement quand le pourcentage de cellules T CD8+ co-exprimant PD-1 et Tim-3 est inférieur à sa valeur de référence prédéterminée correspondante.

5. Procédé selon la revendication 4, dans lequel l'inhibiteur de point de contrôle immunitaire est un anticorps.

6. Procédé selon la revendication 4, dans lequel l'inhibiteur de point de contrôle immunitaire est un anticorps monoclonal.

7. Procédé selon la revendication 4, dans lequel l'inhibiteur de point de contrôle immunitaire est un anticorps anti-PD-1.

8. Procédé selon la revendication 4, dans lequel l'inhibiteur de point de contrôle immunitaire est un anticorps anti-PD-L1.

9. Procédé selon la revendication 4, dans lequel l'inhibiteur de point de contrôle immunitaire est un anticorps anti-PD-L2.

10. Procédé selon la revendication 4, dans lequel l'inhibiteur de point de contrôle immunitaire est un anticorps anti-Tim-3.

11. Procédé selon la revendication 4, dans lequel l'inhibiteur de point de contrôle immunitaire est une petite molécule organique.

12. Procédé selon la revendication 4, dans lequel l'inhibiteur de point de contrôle immunitaire est un aptamère.
